# EUROPEAN PATENT APPLICATION

(11) **EP 2 280 018 A1**
(43) Date of publication of application: **02.02.2011**
(21) Application number: 09735204.1
(22) Date of filing: 22.01.2009
(51) Int. Cl.: C07H 7/027, C07H 1/08

(54) **METHOD FOR EXTRACTING SIALIC ACID-CONTAINING COMPOUND FROM PLANT**

(30) Priority: 25.04.2008 JP 2008115385
(71) Applicant: Incorporated Administrative Agency, National Agriculture and Food Research Organization, Tsukuba-shi, Ibaraki 305-8517 (JP)
(72) Inventor: KAWASE, Shin-ichiro, Fukuyama-shi Hiroshima 7218514 (JP)
(74) Representative: Vossius & Partner
(86) International application number: PCT/JP2009/050974
(87) International publication number: WO 2009/130923

(57) **Abstract**

It is an object of the present invention to provide an extraction method for a sialic acid-containing compound from a natural raw material, which has no risk of contamination with pathogens affecting animals, can achieve mass production, and does not require the use of an organic solvent harmful to the environment and human body. Provided is an extraction method for a water-soluble sialic acid-containing compound, the method comprising: crudely extracting a water-soluble component with water, polyol, or water containing either an acid or an alkali or polyol from a plant body or a processed product of the plant body, in particular, seeds of cereal or seeds of bean; and separating and recovering the water-soluble sialic acid-containing compound from the resultant crude extract solution with a column packed with a serotonin affinity support.

## Description

### Technical Field

The present invention relates to an extraction method for a water-soluble sialic acid-containing compound from a plant, and more specifically, to an extraction method for a water-soluble sialic acid-containing compound from seeds of cereal or seeds of bean.

### Background Art

Sialic acid is a generic term for acyl derivatives of neuraminic acid, which is an amino sugar, and 20 or more types of derivatives are available. Naturally, sialic acid is present as N-acetylneuraminic acid and N-glycolylneuraminic acid in the largest amounts, and is also present in the brains of animals, cow's milk, chicken egg yolk, and the like as "ganglioside", which is sialic acid-containing sphingoglycolipid and as a "polysialic acid sugar chain", which is a sialic acid-containing sugar chain.
Ganglioside is a generic term for glycolipids each having sialic acid at the non-reducing terminal side, and a plurality of types thereof are available depending on the number and binding sites of sialic acids. Polysialic acid is a sugar chain in which several tens or more of sialic acids are bound to each other. Polysialic acid composes a sugar chain moiety of a neural cell adhesion molecule (NCAM) that is a huge nerve specific glycoprotein that functions in the fields of neural cell migration, neurite elongation, and synapse formation.

A compound including sialic acid in its molecular structure has properties of (1) developing a red purple color or a blue purple color by reacting with a resorcinol hydrochloric acid reagent (see e.g., Non-patent Document 1) and (2) exhibiting affinity to a serotonin affinity column (see e.g., Non-patent Document 2). Conversely, a substance that exhibits those properties is a compound having the sialic acid in its molecular structure.

The sialic acid-containing compound is a substance essential for the differentiation, growth, and maintenance of nerves that compose the brain, and the substance has a high functionality. For example, oral ingestion thereof has been observed to enhance learning ability (see e.g., Non-patent Document 3). In addition, the sialic acid-containing compound has been also known to have immunostimulatory effects and anticancer effects. Therefore in the future, the sialic acid-containing compound is expected to be utilized widely for treatments of brain-related diseases and malignant tumor diseases that will increase along with the coming aging society.

The sialic acid-containing compound has been prepared mainly from bovine brain and cow's milk so far, but it has become difficult to prepare from the bovine brain and the cow's milk owing to the occurrence of bovine spongiform encephalopathy (BSE). In addition, a chicken egg is also a source of the sialic acid-containing compound, but safety in the production of the sialic acid-containing compound from the chicken egg has been doubtful owing to the occurrence of avian influenza. That is, it is hard to say that all the consumers agree with its safety, though it has been described that the chicken egg is not infected with an avian influenza virus.
Note that an attempt by a person to produce the sialic acid-containing compound by chemical synthesis has been made. However, the synthetic technique thereof at present has not attained a practical level in terms of yield and cost, and mass production thereof has not been achieved.
From such circumstances, a new source of the sialic acid-containing compound, which has no risk of contamination with pathogens such as a virus or prion which affects animals and is derived from a natural material, has been desired.
In addition, in conventional methods of extracting the sialic acid-containing compound derived from natural substance, the extraction is performed using a chloroform/methanol mixed solvent, hot methanol, or the like (various organic solvents typified by chloroform are also used in chemical synthesis methods of the sialic acid-containing compound). There have also been moves to reduce the use of the harmful organic solvents in light of environmental problems and effects on human bodies, and thus, the development of an extraction method for the sialic acid-containing compound with due consideration to the problems and effects has been desired.

Reports which suggest that sialic acid is present in plants have been described only in the articles of Shar et al. (see Non-patent Documents 4 and 5) and in the article of Richard Bourbouze et al. (see Non-patent Document 6) so far.
However, in the articles of Shar et al., there is only the description of indirect evidence that suggests the presence of sialic acid in *Arabidopsis thaliana,* no experiment for directly examining the presence or absence of the nature inherent to the sialic acid-containing compound has been performed, and no specific extraction method is disclosed.
In addition, in the article of Richard Bourbouze et al., it has been shown only that the extraction of a sugar chain having the affinity to a concanavalin A column (having the nature of exhibiting the affinity to sialic acid but exhibiting the stronger affinity to α-D-mannose and α-D-glucose) was attained, and no experiment for directly examining the presence of the sialic acid-containing compound in the sugar chain has been performed. Further, in the article of Richard Bourbouze et al., only the method in which a glycoprotein soluble in a salt solution is extracted from buckwheat using a buffer, and subsequently the glycoprotein is cut out by hydrolysis and recovered has been shown, and no method by which the glycoprotein can be extracted in a large amount has been disclosed.

Note that, in Patent Document 1, it has been merely described that glycolipids each including ganglioside can be similarly extracted from raw materials derived from living bodies including the plant. However, only the extraction method from murine brain has been disclosed in detail, and no specific extraction method for the glycolipid including ganglioside from the plant as the raw material has been described. In addition, the method is not preferred because chloroform is used.

Non-patent Document 1: Shin Seikagaku Jikken Koza 4 Lipid III Glycolipid, (1990) p149
Non-patent Document 2: Carbohydrate Research, 103, (1982) p213-219
Non-patent Document 3: Ganglioside Kenkyuhou I, (1995), p7-25
Non-patent Document 4: Nature Biotechnology, 21 (2003) p1470-1471
Non-patent Document 5: Nature Biotechnology, 22 (2004) p1351-1352
Non-patent Document 6: Carbohydrate Research, 106, (1982) p21-30
Patent Document 1: JP 2003-129083 A

### Disclosure of the Invention

### Problem to be solved by the Invention

It is an object of the present invention to provide an extraction method for a sialic acid-containing compound from a natural raw material, which has no risk of contamination with pathogens affecting animals, can achieve mass production, and does not require the use of an organic solvent harmful to the environment and human body.

### Means for solving the Problems

As a result of extensive studies for solving the above-mentioned conventional problems, the inventor of the present invention has found that a water-soluble sialic acid compound is present in a plant body, in particular, seeds of cereal and seeds of bean, and has found that a water-soluble sialic acid-containing compound which has no risk of contamination with pathogens affecting animals can be extracted by: crudely extracting a water-soluble component from the plant body with water, polyol, or water containing either an acid or an alkali or polyol; and separating and recovering the water-soluble sialic acid-containing compound from the resultant crude extract solution using a column packed with a serotonin affinity support.
The present invention has been completed based on such findings.

That is, a first aspect of the present invention provides an extraction method for a water-soluble sialic acid-containing compound, the method comprising: crudely extracting a water-soluble component with water, polyol, or water containing either an acid or an alkali or polyol from a plant body or a processed product of the plant body; and separating and recovering the water-soluble sialic acid-containing compound from the resultant crude extract solution with a column packed with a serotonin affinity support.
A second aspect of the present invention provides an extraction method for a water-soluble sialic acid-containing compound according to the first aspect, in which ultrasonication is performed in the step of crudely extracting the water-soluble component with water, the polyol, or water containing either the acid or the alkali or the polyol.
A third aspect of the present invention provides an extraction method for a water-soluble sialic acid-containing compound according to the first or the second aspect, in which ethanol is added to precipitate and remove contaminants after the step of crudely extracting the water-soluble component with water, the polyol, or water containing either the acid or the alkali or the polyol.
A fourth aspect of the present invention provides an extraction method for a water-soluble sialic acid-containing compound according to any one of the first to third aspects, in which the plant body includes seeds of cereal or seeds of bean.
A fifth aspect of the present invention provides a water-soluble sialic acid-containing compound, which is obtained by the extraction method according to any one of the first to fourth aspects.

### Effects of the Invention

According to the present invention, there can be provided an extraction method for a water-soluble sialic acid-containing compound, which has no risk of contamination with pathogens affecting animals, does not require the use of a harmful organic solvent, and can achieve mass production.
According to the present invention, there can be provided a water-soluble sialic acid-containing compound which is safe upon ingestion and is derived from the plant.

### Brief Description of the Drawings

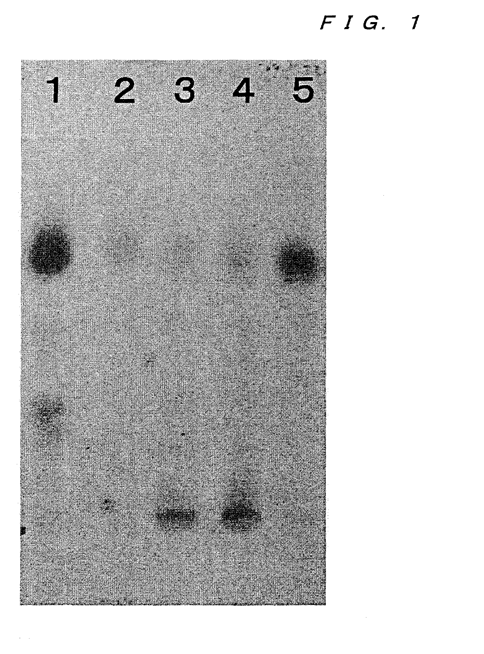

FIG. 1 is an image showing the presence of water-soluble sialic acid-containing compounds detected by a resorcinol hydrochloric acid treatment after crudely extracting from whole soy flour, wheat flour, brown rice whole grain flour, or flour of a brown rice aleurone layer part with water as an extraction solvent, and separating the resultant crude extract solution by thin layer chromatography in Test Examples 1 to 3.

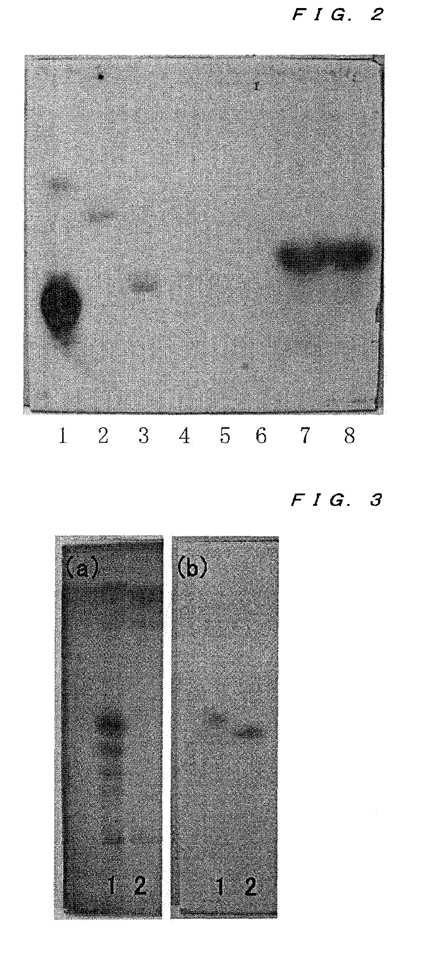

FIG. 2 is an image showing the presence of water-soluble sialic acid-containing compounds detected by the resorcinol hydrochloric acid treatment after crudely extracting from flour of a brown rice aleurone layer part or rice germ with ethylene glycol as the extraction solvent, and separating the resultant crude extract solution by the thin layer chromatography in Test Example 4.
FIG. 3(a) is an image showing the presence of all organic compounds detected under an iodine vapor atmosphere after crudely extracting from flour of a brown rice aleurone layer part with ethylene glycol or water as the extraction solvent, and separating the resultant crude extract solution by the thin layer chromatography in Test Example 5. FIG. 3(b) is an image showing the presence of water-soluble sialic acid-containing compounds detected by the resorcinol hydrochloric acid treatment after separating a crude extract solution extracted from the flour of the brown rice aleurone layer part with ethylene glycol or water as the extraction solvent by the thin layer chromatography in Test Example 5.

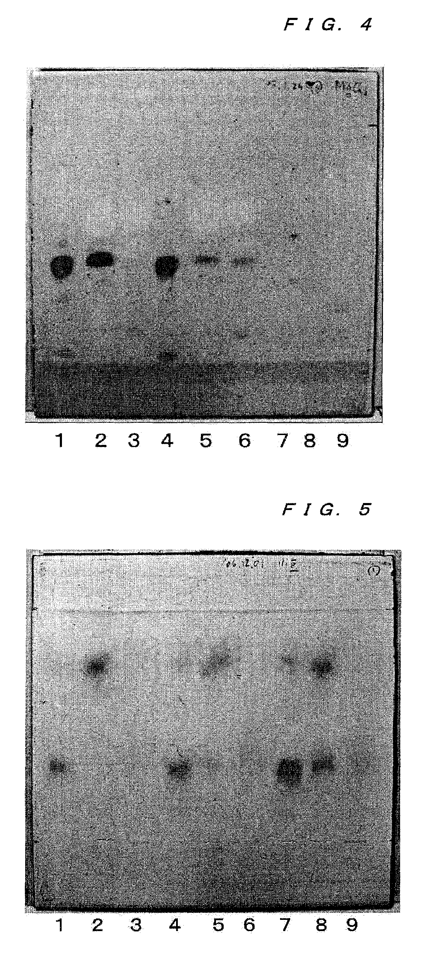

FIG. 4 is an image showing the presence of water-soluble sialic acid-containing compounds detected by the resorcinol hydrochloric acid treatment after crudely extracting from flour of a brown rice aleurone layer part or flour of a polished rice surface layer part with water as the extraction solvent, and separating the resultant crude extract solution by the thin layer chromatography in Test Example 6.
FIG. 5 is an image showing the presence of water-soluble sialic acid-containing compounds detected by the resorcinol hydrochloric acid treatment after crudely extracting from adzuki bean seed flour with water, hydrochloric acid-containing water, or ethylene glycol as the extraction solvent, and separating the resultant crude extract solution by the thin layer chromatography in Test Example 7.

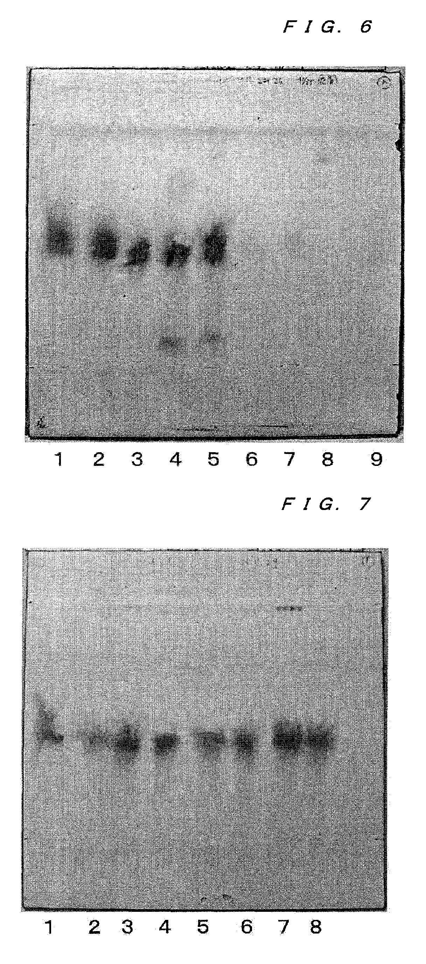

FIG. 6 is an image showing the presence of water-soluble sialic acid-containing compounds detected by the resorcinol hydrochloric acid treatment after crudely extracting from flour of a brown rice outermost layer part, a brown rice aleurone layer part, a polished rice surface layer part, a black soybean seed cotyledon, or corn or maize, and separating the resultant crude extract solution by the thin layer chromatography in Test Examples 8 to 10.
FIG. 7 is an image showing the presence of water-soluble sialic acid-containing compounds detected by the resorcinol hydrochloric acid treatment after crudely extracting from rice germ or flour of a brown rice aleurone layer part with water as the extraction solvent, and separating the resultant crude extract solution by the thin layer chromatography in Test Example 11.

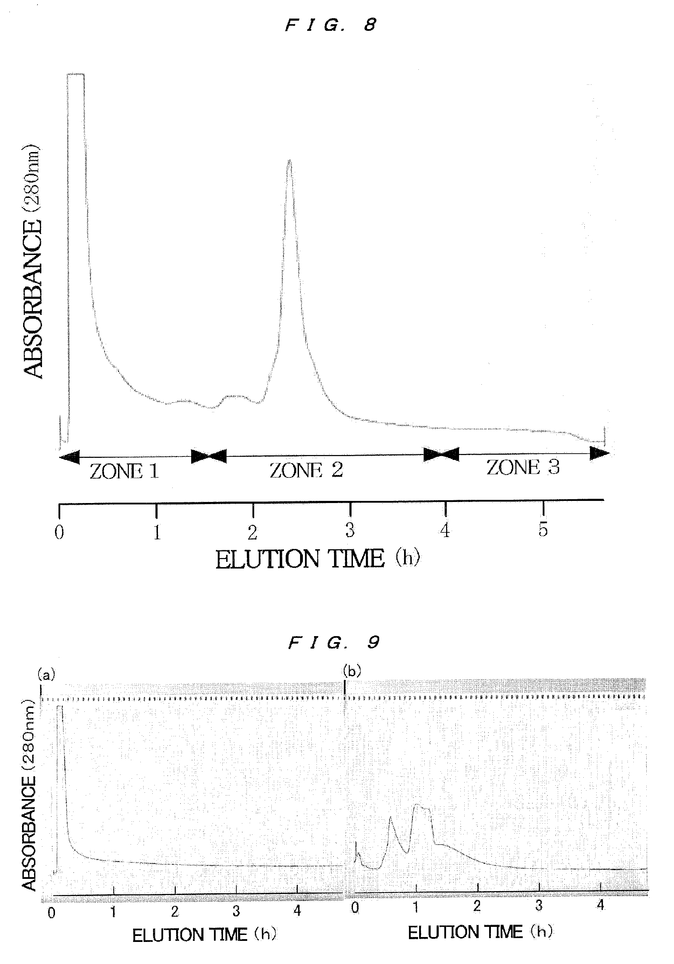

FIG. 8 is a chart in the case of crudely extracting from flour of a brown rice aleurone layer part with water as the extraction solvent, and fractionating the resultant crude extract solution by column chromatography using a serotonin affinity support in Example 1.
FIGS. 9 are charts each in the case of crudely extracting from rice germ with water as the extraction solvent, and fractionating the resultant crude extract solution by column chromatography using a serotonin affinity support in Example 2.

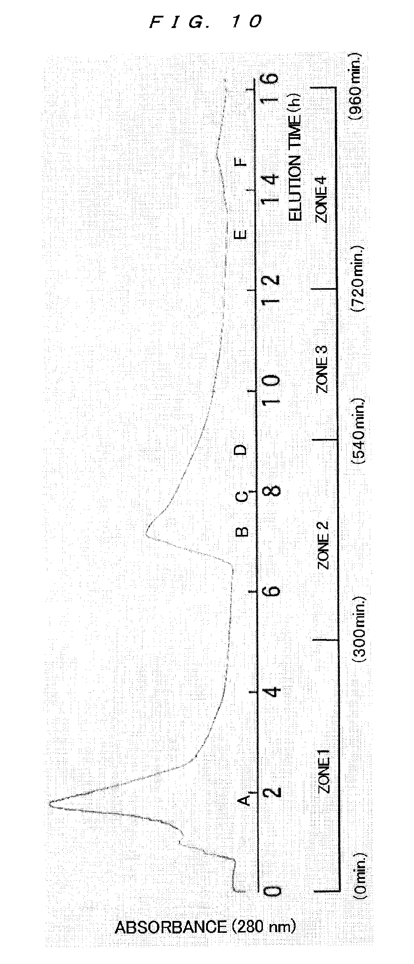

FIG. 10 is a chart in the case of crudely extracting from flour of a polished rice surface layer part with water as the extraction solvent, and fractionating the resultant crude extract solution by column chromatography using a serotonin affinity support in Example 3.

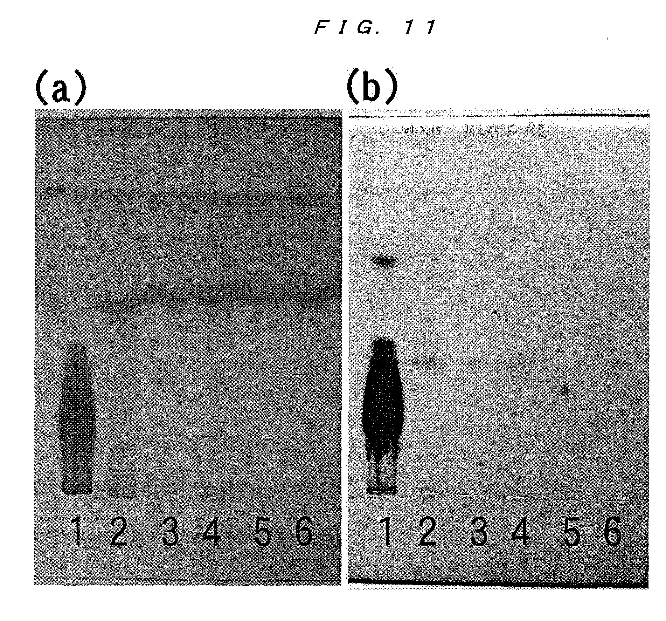

FIG. 11(a) is an image showing the presence of all organic compounds detected under the iodine vapor atmosphere after separating the fraction fractionated with a column packed with a serotonin affinity support by the thin layer chromatography. FIG. 11(b) is an image showing the presence of water-soluble sialic acid-containing compounds detected by the resorcinol hydrochloric acid treatment after separating the fraction fractioned with a column packed with a serotonin affinity support by the thin layer chromatography in Example 3.

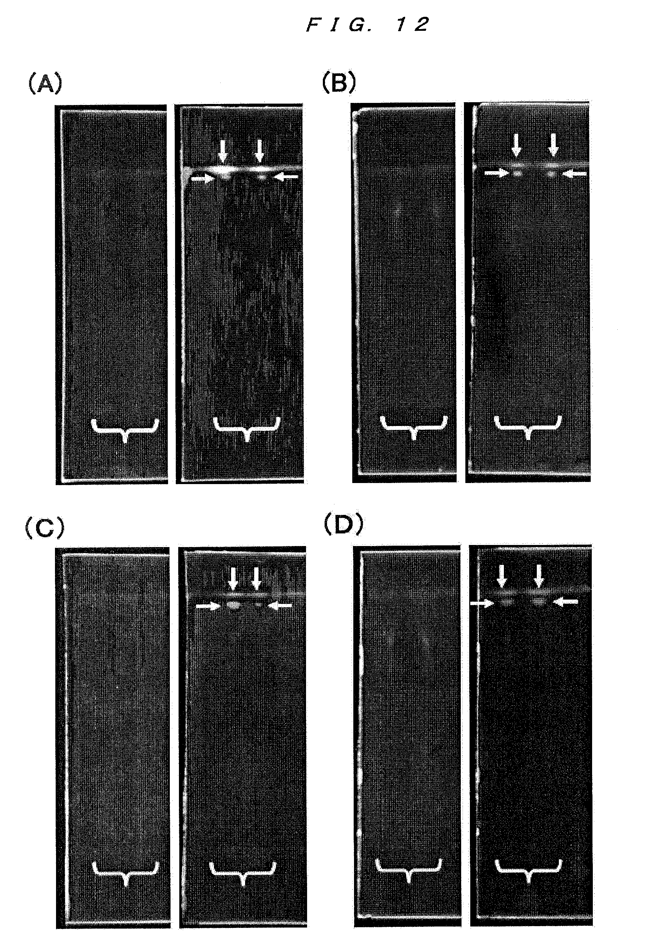

FIGS. 12 are images each showing the presence of sialic acid-containing compounds detected by a lectin binding reaction after crudely extracting from germ with ethylene glycol as the extraction solvent, and separating the resultant crude extract solution by the thin layer chromatography in Test Example 12.

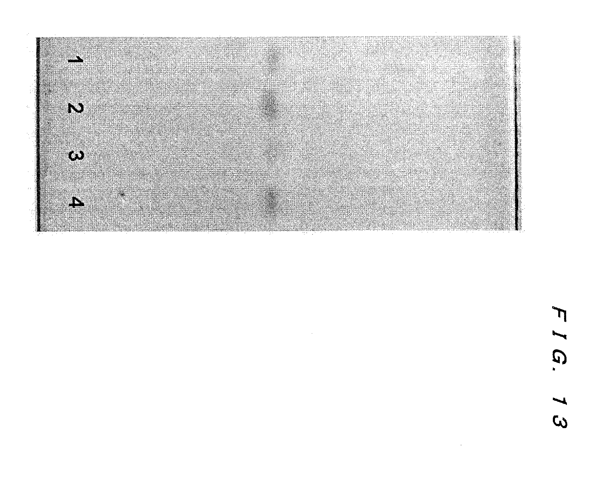

FIG. 13 is an image showing the presence of water-soluble sialic acid-containing compounds detected by the resorcinol hydrochloric acid treatment after crudely extracting from cooked rice excluding a brown rice outermost layer part and including the remaining germ and aleurone layer part with water as the extraction solvent, and separating the resultant crude extract solution by the thin layer chromatography in Test Example 13.

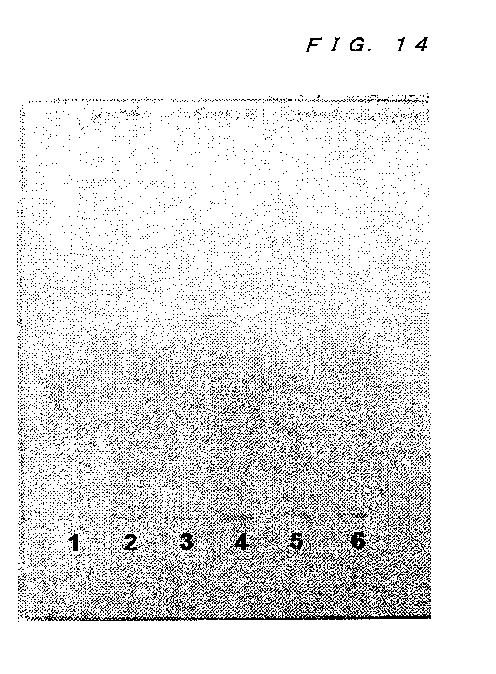

FIG. 14 is an image showing the presence of water-soluble sialic acid-containing compounds detected by the resorcinol hydrochloric acid treatment after crudely extracting from wheat whole grain flour or barley whole grain flour with glycerol as the extraction solvent, and separating the resultant crude extract solution by the thin layer chromatography in Test Example 14.

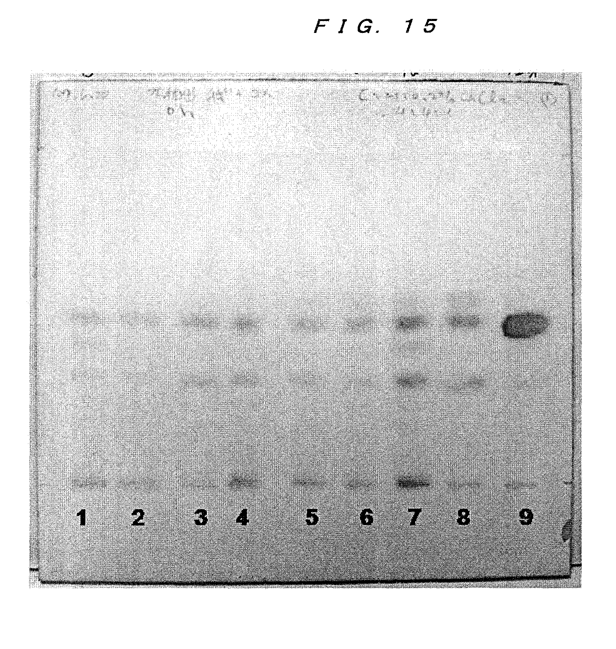

FIG. 15 is an image showing the presence of water-soluble sialic acid-containing compounds detected by the resorcinol hydrochloric acid treatment after crudely extracting from wheat whole grain flour, barley whole grain flour, wheat bran, barley bran, or the rice germ with water as the extraction solvent, and separating the resultant crude extract solution by the thin layer chromatography in Test Example 15.

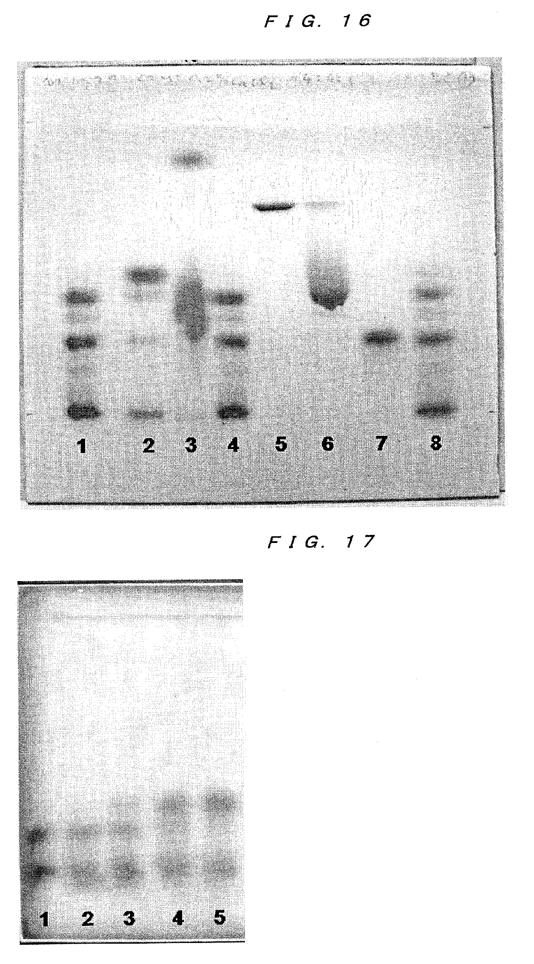

FIG. 16 is an image showing the presence of water-soluble sialic acid-containing compounds detected by the resorcinol hydrochloric acid treatment after crudely extracting from wheat short with water, 11.83% acetic acid, or 7.06% hydrochloric acid as the extraction solvent, and separating the resultant crude extract solution by the thin layer chromatography in Test Example 16.
FIG. 17 is an image showing the presence of water-soluble sialic acid-containing compounds detected by the resorcinol hydrochloric acid treatment after crudely extracting from wheat short with water as the extraction solvent, and separating the resultant crude extract solution by the thin layer chromatography in Test Example 17.

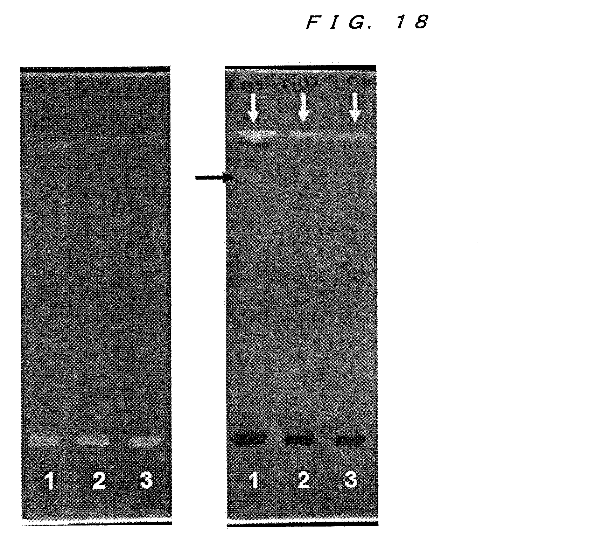

FIG. 18 is an image showing the presence of sialic acid-containing compounds detected by the lectin binding reaction after crudely extracting from rice bran, wheat bran, or barley bran with water as the extraction solvent, and separating the resultant crude extract solution by the thin layer chromatography in Test Example 18.

### Best Modes for carrying out the Invention

The present invention is described in detail below.
The present invention is an extraction method for a water-soluble sialic acid-containing compound, the method comprising: crudely extracting a water-soluble component with water, polyol, or water containing either an acid or an alkali or polyol from a plant body or a processed product of the plant body; and separating and recovering the water-soluble sialic acid-containing compound from the resultant crude extract solution with a column packed with a serotonin affinity support.

The "sialic acid-containing compound" in the present invention refers to a compound having sialic acid in its molecular structure. Sialic acid is a generic term for acyl derivatives of neuraminic acid that is an amino sugar, and 20 or more types of derivatives are available. Naturally, sialic acid is present as N-acetylneuraminic acid and N-glycolylneuraminic acid in the largest amounts. As represented by the structure of N-acetylneuraminic acid of the following chemical formula (1), sialic acid is a nonose having a skeleton comprising nine carbon atoms, and is characterized by having a carboxyl group and an N-acyl group as functional groups.
Note that because of the discovery of 2-keto-3-deoxy-D-glycero-D-galacto-nononic acid (KDN) in which the aminoacyl group at the position 5 has been replaced by a hydroxyl group in 1986, a sugar having an α-keto acid of a nonose skeleton (2-keto-3-deoxynononic acid) is currently employed as the definition of sialic acid.

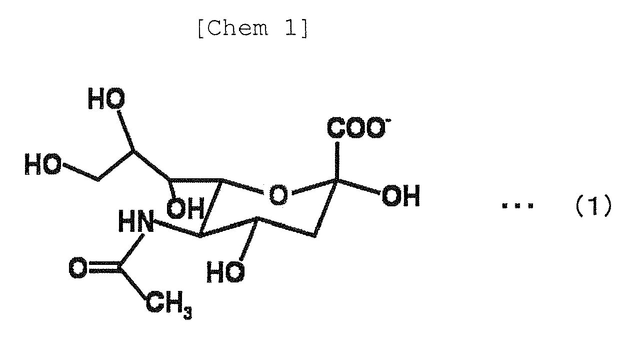

There are given, as molecular species corresponding to the sialic acid-containing compound, a polysialic acid sugar chain which is a sialic acid-containing sugar chain, fetuin, α1-acid glycoprotein and mucin which are sialic acid-containing glycoproteins containing the sugar chain, and ganglioside which is a sialic acid-containing sphingoglycolipid.
Ganglioside is a generic term for glycolipids each having sialic acid at the non-reducing terminal side, and a plurality of types thereof are available depending on the number and binding sites of sialic acids. Polysialic acid is a sugar chain in which several tens or more of sialic acids are bound to each other. Particularly, polysialic acid composes a sugar chain moiety of a neural cell adhesion molecule (NCAM) that is a huge nerve specific glycoprotein that functions in the fields of neural cell migration, neurite elongation, and synapse formation.
In addition, sialic acid has negative charge, is involved in cell adhesion, cell differentiation, and the like, and is particularly a substance essential for the differentiation, growth, and maintenance of the neural cells.
Note that the sialic acid-containing compound in the present invention is a concept encompassing free sialic acid.

Any raw material for the extraction of the sialic acid-containing compound can be used in the present invention as long as the raw material is a plant body, i.e., an organ or a tissue from the plant. The seeds of cereal or seeds of bean are preferably used.
It is desirable to use rice, wheat, soybeans, adzuki beans, corn, barley, or the like as the cereals or beans.
As long as the tissue is derived from the seeds of cereal or seeds of bean, any tissue or part, for example, an embryo, germ, endosperm, seed coat, bran, aleurone layer, starch storage part, or cotyledon may also be used. Further, the entire seeds (whole grains) may also be used.

Further, the processed product of the plant body may be used as the raw material for the extraction of the sialic acid-containing compound in the present invention. Specific examples thereof include wheat flour, rice flour, cooked rice, soybean flour, corn flour, barley flour, adzuki bean flour, coconut powder, and wastes such as rice bran, wheat bran, barley bran, soybean waste, irregular adzuki beans, residue after extracting starch from corn (such as corn germs), sugarcane bagasse, and shochu distillery by-products.
Of those, particularly suitably used are processed products of the cereals or beans such as wheat flour, rice flour, cooked rice, soybean flour, corn flour, barley flour, adzuki bean flour, rice bran, wheat bran, and barley bran.

The water-soluble sialic acid-containing compound of the present invention can be extracted by the following method.
The plant body as the raw material can be directly used for the crude extraction of a water-soluble component depending on the site of the organ or the tissue, but it is desirable to cut finely or crush into a size capable of being used for the crude extraction treatment, and then preferably "mill or pulverize" the plant body as the raw material in terms of enhancement in extraction efficiency followed by using for the crude extraction of the water-soluble component.
The plant body raw material can be milled or pulverized by any method publicly known in the art. Particularly when the cereals are used for the material, the cereals can be pulverized using a mill (Ultracentrifugal Mill manufactured by MRK-RETSCH or Cyclone Sample Mill manufactured by UDY CORPORATION), a brush type rice milling machine (HRG-122 manufactured by Minoru Industry Co., Ltd.), or a grinding type rice milling machine (Grain Testing Mill manufactured by SATAKE). In addition, when the beans are used for the material, a mill (Ultracentrifugal Mill manufactured by MRK-RETSCH) can be used.
Note that rice germ which is dropped out in a rice milling process and powdery processed products such as wheat flour, rice flour, soybean flour, corn flour, barley flour, and coconut powder can be directly used for the crude extraction of the water-soluble component without being milled or pulverized.

First, the water-soluble component is crudely extracted by immersing and mixing the plant body or the processed product of the plant body used as the raw material of the present invention in and with "water", "polyol", or "water containing either an acid or an alkali or polyol".
The water-soluble component is crudely extracted by adding 1 to 10 parts by mass, preferably 3 to 5 parts by mass of the water to 1 part by mass of the plant body or the processed product of the plant body and mixing them well.
Any water such as tap water, distilled water, and deionized water can be used for water used as the extraction solvent in the present invention. Water containing either the acid or the alkali or the polyol can also be used as described above.
In addition, any polyol can be used as the extraction solvent in the present invention as long as the polyol can be used as a "100% polyol solvent", and preferably ethylene glycol and glycerol can be used suitably.

In the present invention, any molecular species of the sialic acid-containing compound can be extracted as long as the sialic acid-containing compound has water solubility. In addition, even if the water solubility is weak or a solubility varies depending on a change in pH, the solubility is enhanced and the extraction can be performed in some cases by using polyol or water in the presence of the acid, the alkali, or the polyol.
Note that it is preferable to use water containing either the acid or the alkali because the growth of various kinds of harmful bacteria during long-term immersion at temperatures ranging from 25 to 40°C can be inhibited. It is also preferable to use polyol or polyol-containing water because contaminants can be reduced.

Examples of the acid which can be contained in water as the extraction solvent in the present invention include hydrochloric acid, sulfuric acid, phosphoric acid, nitric acid, acetic acid, citric acid, and malic acid. Preferably, hydrochloric acid and acetic acid can be suitably used.
Examples of the alkali which can be contained in water as the extraction solvent in the present invention include sodium hydroxide, potassium hydroxide, calcium hydroxide, magnesium hydroxide, and ammonia. Preferably, magnesium hydroxide and ammonia can be suitably used.
Examples of the polyol which can be contained in water as the extraction solvent in the present invention include ethylene glycol, propylene glycol, polyethylene glycol, glycerol (glycerine), polyglycerine, sorbitol, glucose, sucrose, trehalose, and xylitol. Preferably, ethylene glycol and glycerol can be suitably used.

The concentration of "the acid, the alkali, or the polyol which can be contained in water as the extraction solvent" in the present invention varies depending on the substance to be used. For example, when hydrochloric acid is used, it is desirable to incorporate hydrochloric acid at a concentration of 0.5 to 12% (v/v) or preferably about 1% (v/v) to 7% (v/v) (about 0.28 N to 2 N). When acetic acid is used, it is desirable to incorporate acetic acid at a concentration of 0.5 to 12% (v/v) or preferably about 1.7% (v/v) to 12% (v/v) (about 0.28 N to 2 N).
In addition, when ethylene glycol is used, it is desirable to incorporate ethylene glycol at a concentration of 20% (v/v) or more and less than 100% (v/v) or preferably 50% (v/v) or more and less than 100% (v/v) in water.

The plant body or the processed product of the plant body immersed in and mixed with "water", the "polyol", or "water containing either the acid or the alkali or the polyol" can be milled into a finer form by being subjected to ultrasonication for 1 to 30 minutes or preferably 1 to 2 minutes. As a result, the elution efficiency of the water-soluble sialic acid-containing compound can be enhanced.
The ultrasonication in the present invention can be performed by any of the conventionally employed methods, and for example, a B-42J Ultrasonic Cleaner (manufactured by BRANSON) can be used.

Subsequently, the plant body or the processed product of the plant body immersed in and mixed with water, the polyol, or water containing either the acid or the alkali or the polyol can be left to stand still or shaken at a temperature of 1 to 80°C, preferably 5 to 40°C, or more preferably 5 to 10°C for 1 minute to 24 hours or preferably 5 minutes to 12 hours, to thereby enhance the elution efficiency of the water-soluble sialic acid-containing compound.
Note that when one wishes to prevent the change of the sialic acid compound due to enzymatic metabolism, it is desirable that the plant body or the processed product of the plant body be left to stand still or shaken at a temperature of 5 to 10°C for 5 minutes to 12 hours (particularly 5 minutes to 4 hours).
In addition, conversely, in order to enhance the elution efficiency by reducing the molecular weight of the sialic acid-containing compound or fragmenting the sialic acid-containing compound (enhancing easily-soluble property), it is desirable to make an endogenous enzyme contained in the plant body as the raw material act on by leaving to stand still or shaking at room temperature (e.g., about 25°C) for 4 hours or more, preferably 12 hours or more, or more preferably 24 hours or more.
In addition, (when the processed product of a plant body in which the endogenous enzyme has been inactivated is used for the raw material), it is desirable to leave to stand still or shake in water containing an acid at a high concentration (e.g., about 2 N hydrochloric acid or acetic acid) at about 40 to 80°C or preferably at about 80°C.

After the step of the crude extraction, the resultant crude extract solution containing the water-soluble sialic acid-containing compound is separated from a precipitate which is a residue, and recovered. Solid-liquid separation can be performed by any conventional method, and the residue can be separated to recover a filtrate by filtrating through filter paper, fabric, or gauze. It is suitable to perform solid-liquid separation by centrifugation.
When the liquid is separated from the solid by the centrifugation, specifically the crude extract solution can be separated and recovered from the plant residue by centrifuging at 500 to 18,000 rpm for 10 to 60 minutes or preferably at 3000 rpm for about 10 minutes.

The crude extract solution obtained in the above-mentioned step and containing the water-soluble sialic acid-containing compound as a result of the solid-liquid separation can be subjected to a "chromatography treatment" which is a subsequent step, but prior thereto, it is desirable to remove the contaminants contained in the crude extract solution by an ethanol precipitation treatment. Note that, removing the contaminants by the ethanol precipitation treatment can widely reduce the clogging of the column upon performance of the column chromatography in the subsequent step.
The amount of ethanol to be added to the crude extract solution is desirably 0.5 to 5 times as large as, or preferably almost equal to the amount of the crude extract solution. Note that the addition of ethanol in the ethanol precipitation treatment, which refers to a manipulation of adding ethanol to the crude extract solution, includes a manipulation of adding the crude extract solution to ethanol taken in advance.
By the addition of ethanol, ethanol-insoluble components contained in the crude extract solution such as part of nucleic acids, proteins, and polysaccharides form the precipitates as the contaminants.

The solid-liquid separation may be performed immediately after the addition of ethanol, but it is desirable that the solid-liquid separation be performed to recover the crude extract solution after sufficient formation of the precipitates by leaving to stand still preferably at 5 to 40°C for 5 to 24 hours or more preferably 5 to 10°C for 5 to 12 hours.
The solid-liquid separation can be performed by any method, but it is suitable to perform solid-liquid separation by the centrifugation. When the solid-liquid separation is performed by the centrifugation, specifically, the crude extract solution can be separated and recovered from the contaminant precipitates by centrifuging at 500 to 18,000 rpm for 10 to 60 minutes or preferably at 3000 rpm for about 10 minutes.

The sialic acid-containing compound contained in the crude extract solution obtained through the above-mentioned steps can be separated by applying the crude extract solution to the chromatography.
A separation method for fractions containing the sialic acid-containing compound can include thin layer chromatography in which a sample is developed on a flat surface and column chromatography in which a sample is separated using the column. In the thin layer chromatography, a small amount of sample is developed on the flat surface and the presence of an objective substance can be detected semi-quantitatively. However, to separate and recover the objective substance in a large amount from a liquid sample, it is suitable to employ the "column chromatography".

Note that the fraction containing the sialic acid-containing compound can be separated and detected by thin layer chromatography specifically as described below.

### [Detection using resorcinol hydrochloric acid]

The sample can be developed with CH₃Cl/CH₃OH/0.2% CaCl₂=3 to 5/3 to 5/0 to 2 (V/V/V) or preferably 4/4/1 (V/V/V) and separated on a silica gel plate. In addition, the development may be performed using those containing acetone, acetic acid, pyridine, and the like in addition to CH₃Cl, CH₃OH, and CaCl₂.
The fraction containing the sialic acid-containing compound can be detected on the developed silica gel plate by spraying resorcinol hydrochloric acid on the developed plate and heating at 90 to 105°C or preferably 95°C to develop a color.

### [Detection using lectin binding reaction]

Alternatively, the fraction containing the sialic acid-containing compound can be detected on the developed plate by developing and separating with CH₃Cl/CH₃OH/0.2% CaCl₂=3 to 5/3 to 5/1 to 2 (V/V/V) or preferably 4/4/1 (V/V/V) or CH₃Cl/CH₃OH/water=3 to 5/3 to 5/1 to 2 (V/V/V) or preferably 4/4/1 (V/V/V) on the silica gel plate and detecting a substance which is specifically bound to fluorescence-labeled lectin.

Note that the amount of the sample which can be applied to the thin layer chromatography is about 1 to 100 µL (e.g., when a plate size is 20 cm x 20 cm, the amount of the sample which can be applied is about 100 µL), and thus, the amount of the sialic acid-containing compound present in the fraction detected by the color development reaction is an extremely trace amount. It is further required to recover the sialic acid-containing compound from the separated fraction.
Therefore, as described above, the column chromatography is suitable for the purpose of separating and recovering the objective substance in a large amount from the liquid sample.

In the present invention, the column chromatography for separating and recovering the sialic acid-containing compound refers to the column chromatography in which the column support having the strong affinity specific for the sialic acid-containing compound is used and the sialic acid-containing compound can be extremely specifically dissociated by elution, and the sialic acid-containing compound can be separated and recovered by the column chromatography.

A serotonin affinity support can be used as the column support having the strong affinity specific for the sialic acid-containing compound in the present invention. Specifically, the serotonin affinity support (LAS-Serotonin Support manufactured by J-Oil Mills) can also be used. Alternatively, serotonin as a ligand can be chemically coupled to an appropriate commercially available support (e.g., Sephadex manufactured by Pharmacia) to make and use a serotonin affinity support.
Note that an example of the support having an affinity to sialic acid is concanavalin A (ConA) support, which cannot, however, be used as the column support in this step because the ConA support exhibits the stronger affinity to 'α-D-mannose and α-D-glucose' than to sialic acid.

Any scale of the column packed with the serotonin affinity support used for the present invention can be used depending on the amount of the extract solution, and its scale can be increased if necessary. In addition, the multiple columns can also be prepared and run concurrently for the purification. Specifically when 0.1 mL of the crude extract solution is applied, a column with a diameter of 4 mm and a length of 5 cm or a column with a diameter of 4.6 mm and a length of 15 cm can be used. In addition, when 2 mL of the crude extract solution is applied, a column with a diameter of 26 mm and a length of 4.6 cm can be used.

In the column chromatography in the present invention, first the crude extract solution is applied to the column packed with the serotonin affinity support and adsorbed to the serotonin affinity support. The water-soluble sialic acid-containing compound in the water-soluble components contained in the applied crude extract solution is adsorbed to the serotonin affinity support in the range of an adsorption allowable volume of the support.
Subsequently, unadsorbed contaminants which have not been adsorbed to the adsorption support are removed by running water to wash the adsorption column. Water is desirably run under conditions of a flow rate of 0.1 to 0.5 mL/min or preferably 0.2 mL/min and a running time of water of 90 to 600 minutes or preferably 90 to 300 minutes.
Note that any water such as distilled water, tap water, deionized water, and pure water can be used as water used here, and specifically pure water can be used.

In addition, to assure an interaction between the sialic acid-containing compound and the serotonin affinity support, leaving stand for 5 to 72 hours or preferably about 12 hours after running water can assure the interaction between the sialic acid-containing compound and the serotonin affinity support.

Subsequently, the water-soluble sialic acid-containing compound can be dissociated and recovered from the serotonin affinity support by running an ammonium acetate solution or a sodium chloride solution, and specifically the ammonium acetate solution can be used. Using a 0.1 to 100 mM or preferably 4 to 30 mM ammonium acetate solution, the solution is desirably run under conditions of a flow rate of 0.1 to 0.5 mL/min or preferably 0.2 mL/min, and a running time of the solution of 120 to 420 minutes or preferably 150 to 300 minutes.
In addition, the ammonium acetate solution with a linear gradient from 0 M to 2 M or preferably 0 M to 1 M may also be run.
By the above-mentioned step, a fraction of the water-soluble sialic acid-containing compound eluted in the ammonium acetate solution can be obtained.
Note that, after the fraction of the water-soluble sialic acid-containing compound has been recovered, the column can be washed by running a 0.1 to 1 M ammonium acetate solution containing 0 to 1 M sodium chloride for 60 to 300 minutes and then running pure water for 60 to 300 minutes.

A diluted ammonium acetate solution which is the fraction of the water-soluble sialic acid-containing compound recovered through the above-mentioned step can also be used for the raw materials of foods and pharmaceuticals as a liquid after the removal of an ammonium salt by volatilization or dialysis, and a dry solid or dry powder thereof can also be used.
Here, the dry solid or dry powder of the fraction of the water-soluble sialic acid-containing compound can be obtained by methods such as an evaporator, freeze-drying, nitrogen gas flow drying, and reduced pressure drying. Specifically, it is desirable to completely dry using Freezdryer FD-1 (manufactured by EYELA) or Dry Thermo Bath MG-2100 (manufactured by EYELA). Alternatively, an appropriate amount of ethanol may be added to completely vaporize the solvent.
Note that it is also possible to make the dry solid or the dry powder by a technique such as the freeze-drying or the nitrogen gas flow drying and dissolve in a solvent such as water when needed.

Concerning the amount of the water-soluble sialic acid-containing compound recovered through the above-mentioned steps, when 3 g of a plant as the raw material, specifically the seeds of cereal or seeds of bean are used, about 2.5 to 4.5 mg of the water-soluble sialic acid-containing compound can be extracted.
In addition, the content of the contaminants in the fraction of the water-soluble sialic acid-containing compound recovered through the above-mentioned steps is not more than the detection limit of an all organic compound amount detected under the iodine vapor atmosphere, and thus, the sialic acid-containing compound in larger amount with much higher purity compared with the prior art can be extracted in the present invention.

### Examples

The present invention is described in more detail by examples, but the scope of the present invention is by no means limited by these examples.

### Test Example 1 (Detection of sialic acid-containing compounds from crude extract solution of soybean seeds)

15 mL of water were added to 3 g of soybean flour obtained by milling whole soy (Sachiyutaka) with a mill (Ultracentrifugal Mill manufactured by MRK-RETSCH, mesh diameter: 0.5 mm or 1 mm), and the contents were mixed well (sample 1-1). The mixture was subjected to ultrasonication using an ultrasonic apparatus (B-42J Ultrasonic Cleaner manufactured by BRANSON) for 1 minute and 30 seconds. The treated product was left to stand still at 10°C for 12 hours and then centrifuged at 3000 rpm for 10 minutes to perform solid-liquid separation.
An equal amount of ethanol was added to the separated crude extract solution, and the contents were mixed well. After that, the mixture was left to stand still at 10°C for 12 hours and then centrifuged at 3000 rpm for 10 minutes to perform solid-liquid separation.
The separated supernatant was developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 1 shows the results.

### Test Example 2 (Detection of sialic acid-containing compounds from crude extract solutions of brown rice whole grain and brown rice aleurone layer part)

15 mL of water were added to 3 g of flour obtained by milling brown rice (Koshihikari) whole grains with a mill (Cyclone Sample Mill manufactured by UDY Corporation), and the contents were mixed well (sample 2-2).
Further, 15 mL of water were added to 3 g of flour derived from a part in the range of 96 to 91% by mass corresponding to an aleurone layer part when brown rice was regarded as 100% by mass (brown rice aleurone layer part having a volume of 5 % by mass), the part being obtained by grinding out brown rice (Koshihikari) successively from the outside using a grinding type rice milling machine (Satake Grain Testing Mill manufactured by Satake), and the contents were mixed well (sample 2-5).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.
The separated supernatants were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 1 shows the results.

### Test Example 3 (Detection of sialic acid-containing compounds from crude extract solution of wheat flour)

15 mL of water were added to 3 g of wheat flour (Norin 61), and the contents were mixed well (sample 3-3). 15 mL of water were added to 3 g of wheat flour (Western White), and the contents were mixed well (sample 3-4).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.
The separated supernatants were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 1 shows the results.

Note that, in FIG. 1, the crude extracts obtained using water as the extraction solvent in the above-mentioned steps were developed on a silica gel plate, wherein lane 1 shows that from the whole soy (Sachiyutaka) flour (sample 1-1), lane 2 shows that from the brown rice (Koshihikari) whole grain flour (sample 2-2), lane 3 shows that from the wheat flour (Norin 61) (sample 3-3), lane 4 shows that from the wheat flour (Western White) (sample 3-4), and lane 5 shows that from the flour of the brown rice (Koshihikari) aleurone layer part (sample 2-5), respectively.

As shown in FIG. 1, it was demonstrated that the water-soluble sialic acid-containing compound was contained in each of the crude extract solutions obtained in the above-mentioned steps using water as the extraction solvent and using the whole soy flour (sample 1-1) (lane 1), the brown rice whole grain flour (sample 2-2) (lane 2), the wheat flours (samples 3-3, 3-4) (lane 3, 4), and the flour of the brown rice aleurone layer part (sample 2-5) (lane 5) as the raw materials. It was also shown that the water-soluble sialic acid-containing compound capable of being extracted by the above-mentioned method was contained particularly in abundance in each of the whole soy flour (sample 1-1) (lane 1) and the flour of the brown rice aleurone layer part (sample 2-5) (lane 5) among those samples.
The results revealed that the crude extract solution containing the water-soluble sialic acid-containing compound was obtained from each of the flours derived from the seeds of the soybean, the rice, and the wheat by using water as the extraction solvent without using a harmful organic solvent.

### Test Example 4 (Detection of sialic acid-containing compounds from crude extraction solution using ethylene glycol)

15 mL of 100% (v/v) ethylene glycol were added to 3 g of flour derived from a part in the range of 96 to 91% by mass corresponding to an aleurone layer part when brown rice was regarded as 100% by mass (brown rice aleurone layer part having a volume of 5 % by mass), the part being obtained by grinding out brown rice (Koshihikari) successively from the outside using a grinding type rice milling machine (Grain Testing Mill manufactured by SATAKE), and the contents were mixed well (sample 4-7). In addition, 15 mL of 100% (v/v) ethylene glycol were added to 3 g of rice germ (Koshihikari), and the contents were mixed well (sample 4-8).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
The separated supernatants were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 2 shows the results.

Note that, in FIG. 2, lane 1 is authentic N-acetylneuraminic acid (containing sialic acid) (N-Acetylneuraminic acid Type IV-S, manufactured by SIGMA), lane 2 is authentic ganglioside G_{D1a} (containing sialic acid) (Disialoganglioside-G_{D1a}, manufactured by SIGMA), lane 3 is authentic ganglioside G_{T1b} (containing sialic acid) (Trisialoganglioside-G_{T1b,} manufactured by SIGMA), lane 4 is authentic phosphatidylserine (Phosphatidylserine from beef brain, manufactured by SERDANY Research Laboratories), lane 5 is authentic phosphatidylethanolamine (L-α-Phosphatidylethanolamine from plant, manufactured by AVANTI Polar-Lipids), and lane 6 is authentic lysophosphatidylcholine (L-α-Lysophosphatidylcholine from soybean, manufactured by SIGMA), which were respectively developed on the silica gel plate.
The result of development of the crude extract solution on a silica gel plate obtained in the above-mentioned step by using 100 % (v/v) ethylene glycol (polyol) as the extraction solvent from the flour of the brown rice (Koshihikari) aleurone layer part (sample 4-7) is shown in lane 7 and that from the rice germ (Koshihikari) (sample 4-8) is shown in lane 8, respectively.

As shown in FIG. 2, it was shown that the water-soluble sialic acid-containing compound was also contained in the crude extract solution obtained in the above-mentioned steps by using 100% (v/v) ethylene glycol (polyol) as the extraction solvent and using the flour of the brown rice aleurone layer part (sample 4-7) (lane 7) or the rice germ (sample 4-8) (lane 8) as the raw material.
It was also shown that the water-soluble sialic acid-containing compound was contained in the rice germ part (sample 4-8) (lane 8) in abundance equal to the brown rice aleurone layer part (sample 4-7) (lane 7).

### Test Example 5 (Influence of ethylene glycol on extraction)

15 mL of water were added to 3 g of flour derived from a part in the range of 96 to 91% by mass corresponding to an aleurone layer part when brown rice was regarded as 100% by mass (brown rice aleurone layer part having a volume of 5 % by mass), the part being obtained by grinding out brown rice (Koshihikari) successively from the outside using a grinding type rice milling machine (Satake Grain Testing Mill manufactured by SATAKE), and the contents were mixed well (sample 5-1). In addition, 15 mL of 100% (v/v) ethylene glycol were added to 3 g of the flour of the brown rice aleurone layer part, and the contents were mixed well (sample 5-2).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.
The separated supernatants were developed with CH₃Cl/CH₃OH/0.2% CaCl₂=4/4/1 (V/V/V) and separated into individual components on a silica gel plate.
The silica gel plate on which the supernatants had been developed was left to stand still under an iodine vapor atmosphere at room temperature for 72 hours, to thereby detect all organic compounds. Subsequently, the silica gel plate taken out of the iodine vapor atmosphere was left to stand still for 24 hours to desorb iodine sufficiently, sprayed with resorcinol hydrochloric acid, and heated at 95°C to develop a color, to thereby detect sialic acid-containing compounds. FIGS. 3 show the results. Note that, in FIGS. 3, FIG. 3 (a) shows the all organic compounds detected and FIG. 3(b) shows the sialic acid-containing compounds detected.

Note that, in FIG. 3, each lane shows a development on a slica gel plate, wherein lane 1 shows that of the crude extract solution (sample 5-1) obtained from the flour of the brown rice aleurone layer part using water as the extraction solvent in the above-mentioned steps and lane 2 shows that of the crude extract solution (sample 5-2) obtained from the flour of the brown rice aleurone layer part using 100% (v/v) ethylene glycol as the extraction solvent in the above-mentioned steps.

The results of the comparison between FIG. 3(a) and FIG. 3(b) showed that the extraction (sample 5-2) (lane 2) of the flour of the brown rice aleurone layer part using 100% (v/v) ethylene glycol as the extraction solvent provided a crude extract solution containing the water-soluble sialic acid-containing compound and less contaminants than the extraction (sample 5-1) (lane 1) using water as the extraction solvent.

### Test Example 6 (Influence of ethanol precipitation on extraction)

15 mL of water were added to 3 g of flour derived from a part in the range of 96 to 91% by mass corresponding to an aleurone layer part when brown rice was regarded as 100% by mass (brown rice aleurone layer part having a volume of 5 % by mass), the part being obtained by grinding out brown rice (Koshihikari) successively from the outside using a grinding type rice milling machine (Grain Testing Mill manufactured by SATAKE), and the contents were mixed well (samples 6-1 to 6-3). Further, 15 mL of water were added to 3 g of flour derived from a part (polished rice surface layer part) in the range of 91 to 86% by mass corresponding to a polished rice surface layer part when brown rice was regarded as 100% by mass, the part being obtained by grinding out brown rice (Koshihikari) successively from the outside using the grinding type rice milling machine (Grain Testing Mill manufactured by SATAKE), and the contents were mixed well (samples 6-4 to 6-6).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.

Concerning the samples obtained in the above-mentioned steps from the flour of the brown rice aleurone layer part and from the flour of the polished rice surface layer part, "centrifuged supernatants after the addition of ethanol" were designated as the samples 6-1 and 6-4, "solutions obtained by redissolving centrifuged precipitates in water after the addition of ethanol" were designated as the samples 6-2 and 6-5, and "solutions obtained by redissolving centrifuged precipitates in water after the first crude extraction with water" were designated as the samples 6-3 and 6-6, and those samples were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 4 shows the results.

Note that, in FIG. 4, each lane shows a development on a slica gel plate, wherein lane 1 shows that of the centrifuged supernatant after the addition of ethanol obtained from the flour of the brown rice aleurone layer part (sample 6-1), lane 2 shows that of the solution obtained from the flour of the brown rice aleurone layer part by redissolving the centrifuged precipitate in water after the addition of ethanol (sample 6-2), lane 3 shows that of the solution obtained from the flour of the brown rice aleurone layer part by redissolving the centrifuged precipitate in water after the first crude extraction with water (sample 6-3), lane 4 shows that of the centrifuged supernatant after the addition of ethanol obtained from the flour of the polished rice surface layer part (sample 6-4), lane 5 shows that of the solution obtained from the flour of the polished rice surface layer part by redissolving the centrifuged precipitate in water after the addition of ethanol (sample 6-5), and lane 6 shows that of the solution obtained from the flour of the polished rice surface layer part by redissolving the centrifuged precipitate in water after the first crude extraction with water (sample 6-6), respectively.
Further, lane 7 is authentic ganglioside G_{D1a} (containing sialic acid) (Disialoganglioside-G_{D1a}, manufactured by SIGMA), lane 8 is authentic ganglioside G_{T1b} (containing sialic acid) (Trisialoganglioside-G_{T1b}, manufactured by SIGMA), and lane 9 is authentic N-acetylneuraminic acid (containing sialic acid) (N-Acetylneuraminic acid Type IV-S, manufactured by SIGMA), which were respectively developed on the silica gel plate.

As shown in FIG. 4, it was shown that the water-soluble sialic acid-containing compound was contained particularly in abundance in each of the centrifuged supernatants (crude extract solution) (samples 6-1, 6-4) (lanes 1 and 4) after the addition of ethanol obtained from the flour of the brown rice aleurone layer part and the polished rice surface layer part by the above-mentioned extraction method.
In addition, when the flour of the brown rice aleurone layer part was used as the raw material, it was also shown that the water-soluble sialic acid-containing compound was contained in abundance in the solution obtained by redissolving the centrifuged precipitate in water after the addition of ethanol (sample 6-2) (lane 2).
Note that the water-soluble sialic acid-containing compound was scarcely contained in each of the solutions (samples 6-3 and 6-6) (lanes 3 and 6) obtained from the flour of the brown rice aleurone layer part and the polished rice surface layer part by redissolving the centrifuged precipitate (residue) in water after the first crude extraction with water, and thus, it was shown that most of the water-soluble sialic acid-containing compound had been extracted into the crude extract solution in the first crude extraction with water.

### Test Example 7 (Detection of sialic acid-containing compounds from crude extract solution using various solvents)

15 mL of water were added to 3 g of flour obtained by milling adzuki bean seeds with a mill (Ultracentrifugal Mill manufactured by MRK-RETSCH), and the contents were mixed well (samples 7-1, 7-4, and 7-7). In addition, 15 mL of 1% (v/v) hydrochloric acid were added to 3 g of the adzuki bean seed flour, and the contents were mixed well (samples 7-2, 7-5, and 7-8). In addition, 15 mL of 100% (v/v) ethylene glycol were added to 3 g of the adzuki bean seed flour, and the contents were mixed well (samples 7-3, 7-6, and 7-9).
Those mixtures were subjected to ultrasonication using an ultrasonic apparatus (B-42J Ultrasonic Cleaner manufactured by BRANSON) for 1 minute and 30 seconds. Those treated products were left to stand still at 10°C (samples 7-7 to 7-9), 25°C (samples 7-4 to 7-6), or 37°C (samples 7-1 to 7-3) for 12 hours, and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.
The separated supernatants were developed with CH₃Cl/CH₃OH/0.2% CaCl₂=4/4/1 (v/v/v) and separated into individual components on a silica gel plate. The silica gel plate was sprayed with resorcinol hydrochloric acid and heated at 95°C to develop a color, to thereby detect sialic acid-containing compounds. FIG. 5 shows the results.

Note that, in FIG. 5, each lane shows a development on a slica gel plate, wherein lane 1 shows that of the crude extract solution obtained from the adzuki bean seed flour using water at 37°C as the extraction solvent in the above-mentioned steps (sample 7-1), lane 2 shows that of the crude extract solution obtained from the adzuki bean seed flour using 1% (v/v) hydrochloric acid at 37°C as the extraction solvent in the above-mentioned steps (sample 7-2), lane 3 shows that of the crude extract solution obtained from the adzuki bean seed flour using 100% (v/v) ethylene glycol at 37°C as the extraction solvent in the above-mentioned steps (sample 7-3), lane 4 shows that of the crude extract solution obtained from the adzuki bean seed flour using water at 25°C as the extraction solvent in the above-mentioned steps (sample 7-4), lane 5 shows that of the crude extract solution obtained from the adzuki bean seed flour using 1% (v/v) hydrochloric acid at 25°C as the extraction solvent in the above-mentioned steps (sample 7-5), lane 6 shows that of the crude extract solution obtained from the adzuki bean seed flour using 100% (v/v) ethylene glycol at 25°C as the extraction solvent in the above-mentioned steps (sample 7-6), lane 7 shows that of the crude extract solution obtained from the adzuki bean seed flour using water at 10°C as the extraction solvent in the above-mentioned steps (sample 7-7), lane 8 shows that of the crude extract solution obtained from the adzuki bean seed flour using 1% (v/v) hydrochloric acid at 10°C as the extraction solvent in the above-mentioned steps (sample 7-8), and lane 9 shows that of the crude extract solution obtained from the adzuki bean seed flour using 100% (v/v) ethylene glycol at 10°C as the extraction solvent in the above-mentioned steps (sample 7-9), respectively.

As shown in FIG. 5, it was shown that the crude extract solution containing the water-soluble sialic acid-containing compound was also obtained from the flour of the adzuki bean seeds by extraction using water, 1% (v/v) hydrochloric acid, or 100% (v/v) ethylene glycol as the extraction solvent at a temperature of 10, 25, or 37°C.

### Test Example 8 (Detection of sialic acid-containing compounds from crude extract solution from each layer of brown rice)

15 mL of water were added to 3 g of flour derived from a part in the range of 100 to 96% by mass corresponding to an outermost layer part when brown rice was regarded as 100% by mass (brown rice outermost layer part having a volume of 4% by mass), the part being obtained by grinding out brown rice (Koshihikari) successively from the outside using a grinding type rice milling machine (Grain Testing Mill manufactured by SATAKE), and the contents were mixed well (sample 8-1). Further, 15 mL of water was added to 3 g of flour derived from a part (brown rice aleurone layer part) in the range of 96 to 91% by mass corresponding to an aleurone layer part when brown rice was regarded as 100% by mass, the part being obtained by grinding out brown rice (Koshihikari) successively from the outside using the grinding type rice milling machine (Grain Testing Mill manufactured by SATAKE), and the contents were mixed well (sample 8-2). In addition, 15 mL of water were added to 3 g of flour derived from a part in the range of 91 to 86% by mass corresponding to a polished rice surface layer part when brown rice was regarded as 100% by mass (polished rice surface layer part having a volume of 5% by mass), the part being obtained by grinding out brown rice (Koshihikari) successively from the outside using the grinding type rice milling machine (Grain Testing Mill manufactured by SATAKE), and the contents were mixed well (sample 8-3).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.
The separated supernatants were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 6 shows the results.

### Test Example 9 (Detection of sialic acid-containing compounds from crude extract solution of dehulled black soybean seed cotyledon part)

15 mL of water were added to 3 g of flour obtained by milling a cotyledon part of dehulled black soybean seeds with a mill (Ultracentrifugal Mill manufactured by MRK-RETSCH), and the contents were mixed well (sample 9-4). Further, 15 mL of 1% (v/v) hydrochloric acid were added to 3 g of the flour of the cotyledon part of dehulled black soybean seeds, and the contents were mixed well (sample 9-5).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.
The separated supernatants were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 6 shows the results.

### Test Example 10 (Detection of sialic acid-containing compounds from crude extract solution of corn seeds)

15 mL of water were added to 3 g of flour obtained by milling corn seeds (popcorn seeds) with a mill (Ultracentrifugal Mill manufactured by MRK-RETSCH), and the contents were mixed well (sample 10-6). Further, 15 mL of 1% (v/v) hydrochloric acid were added to 3 g of the flour of the corn seed (popcorn seeds), and the contents were mixed well (sample 10-7).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.
The separated supernatants were developed with CH₃C1/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 6 shows the results.

Note that, in FIG. 6, each lane shows a development on a slica gel plate, wherein lane 1 shows that of the crude extract solution obtained from the flour of the brown rice outermost layer part using water as the extraction solvent in the above-mentioned steps (sample 8-1), lane 2 shows that of the crude extract solution obtained from the flour of the brown rice aleurone layer part using water as the extraction solvent in the above-mentioned steps (sample 8-2), lane 3 shows that of the crude extract solution obtained from the flour of the polished rice surface layer part using water as the extraction solvent in the above-mentioned steps (sample 8-3), lane 4 shows that of the crude extract solution obtained from the flour of the cotyledon part of the black soybean seed using water as the extraction solvent in the above-mentioned steps (sample 9-4), lane 5 shows that of the crude extract solution obtained from the flour of the cotyledon part of the black soybean seed using 1% (v/v) hydrochloric acid as the extraction solvent in the above-mentioned steps (sample 9-5), lane 6 shows that of the crude extract solution obtained from the corn seeds using water as the extraction solvent in the above-mentioned steps (sample 10-6), and lane 7 shows that of the crude extract solution obtained from the corn seeds using 1% (v/v) hydrochloric acid as the extraction solvent in the above-mentioned steps (sample 10-7), respectively.
Further, lane 8 is authentic ganglioside G_{D1a} (containing sialic acid) (Disialoganglioside-G_{D1a}, manufactured by SIGMA) and lane 9 is authentic ganglioside G_{T1b} (containing sialic acid) (Trisialoganglioside-G_{T1b}, manufactured by SIGMA), which were respectively developed on the silica gel plate.

As shown in FIG. 6, it was shown that the water-soluble sialic acid-containing compound was also contained in the crude extract solution (sample 8-1) (lane 1) obtained from the flour of the brown rice outermost layer part using water as the extraction solvent in the above-mentioned steps in abundance equal to the crude extract solution (sample 8-2 or 8-3) (lane 2 or 3) obtained from the flour of the brown rice aleurone layer part or the flour of the polished rice surface layer part using water as the extraction solvent in the above-mentioned steps.
It was also shown that the water-soluble sialic acid-containing compound was also contained in the crude extract solution (sample 9-4 or 9-5) (lane 4 or 5) obtained from the flour of the black soybean cotyledon using water or 1% (v/v) hydrochloric acid as the extraction solvent in the above-mentioned steps in abundance equal to the crude extract solution (sample 8-2 or 8-3) (lane 2 or 3) obtained from the flour of the brown rice aleurone layer part or the flour of the polished rice surface layer part using water as the extraction solvent in the above-mentioned steps.
It was also shown that the water-soluble sialic acid-containing compound was also contained in the crude extract solution (sample 10-6 or 10-7) (lane 6 or 7) obtained from the flour of the corn using water or 1% (v/v) hydrochloric acid as the extraction solvent in the above-mentioned steps.

### Test Example 11 (Influence of extraction time on extraction)

15 mL of water were added to 3 g of the rice germ (Koshihikari), and the contents were mixed well (samples 11-1, 11-2, 11-5, and 11-6). Further, 15 mL of water were added to 3 g of flour derived from a part in range of 96 to 91% by mass corresponding to an aleurone layer part when brown rice was regarded as 100% by mass (brown rice aleurone layer part having a volume of 5 % by mass), the part being obtained by grinding out brown rice (Koshihikari) successively from the outside using a grinding type rice milling machine (Grain Testing Mill manufactured by SATAKE), and the contents were mixed well (samples 11-3, 11-4, 11-7, and 11-8).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 2.5 hours (samples 11-1 to 11-4) or 10 hours (samples 11-5 to 11-8) and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.

A "centrifuged supernatant after the addition of ethanol" obtained from the rice germ or from the flour of the brown rice aleurone layer part in the above-mentioned steps was designated as a sample 11-2, 11-4, 11-6, or 11-8, and a "centrifuged supernatant after the first crude extraction with water" was designated as a sample 11-1, 11-3, 11-5, or 11-7, and those samples were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 7 shows the results.

Note that, in FIG. 7, each lane shows a development on a slica gel plate, wherein lane 1 shows that of the centrifuged supernatant after the extraction from the rice germ with water for 2.5 hours (sample 11-1), lane 2 shows that of the centrifuged supernatant after the addition of ethanol obtained in the above-mentioned steps after the extraction from the rice germ with water for 2.5 hours (sample 11-2), lane 3 shows that of the centrifuged supernatant after the extraction from the flour of the brown rice aleurone layer part with water for 2.5 hours (sample 11-3), lane 4 shows that of the centrifuged supernatant after the addition of ethanol obtained in the above-mentioned steps after the extraction from the flour of the brown rice aleurone layer part with water for 2.5 hours (sample 11-4), lane 5 shows that of the centrifuged supernatant after the extraction from the rice germ with water for 10 hours (sample 11-5), lane 6 shows that of the centrifuged supernatant after the addition of ethanol obtained in the above-mentioned steps after the extraction from the rice germ with water for 10 hours (sample 11-6), lane 7 shows that of the centrifuged supernatant after the extraction from the flour of the brown rice aleurone layer part with water for 10 hours (sample 11-7), and the lane 8 shows that of the centrifuged supernatant after the addition of ethanol obtained in the above-mentioned steps after the extraction from the flour of the brown rice aleurone layer part with water for 10 hours (sample 11-8) in lane 8, respectively.

As shown in FIG. 7, it was shown that the water-soluble sialic acid-containing compound was contained in abundance in the crude extract solution obtained by extracting from the rice germ or the flour of the brown rice aleurone layer part with water at 10°C for 2.5 or 10 hours. It was also shown that the amount of the water-soluble sialic acid-containing compound capable of being extracted with water for 10 hours (samples 11-5 to 11-8) (lanes 5 to 8) was increased compared with the case of extraction for 2.5 hours (samples 11-1 to 11-4) (lanes 1 to 4).
Note that, no large difference in extracted amount of the water-soluble sialic acid-containing compound was observed between the centrifuged supernatants after the extraction with water (samples 11-1, 11-3, 11-5, and 11-7) (lanes 1, 3, 5, and 7) and the centrifuged supernatants after the addition of ethanol (samples 11-2, 11-4, 11-6, and 11-8) (lanes 2, 4, 6, and 8).

### Example 1 (Extraction and recovery of sialic acid-containing compounds from brown rice aleurone layer part)

In the same manner as in Test Example 2, 15 mL of water were added to 3 g of flour derived from a part in the range of 96 to 91% by mass corresponding to an aleurone layer part when brown rice was regarded as 100% by mass (brown rice aleurone layer part having a volume of 5 % by mass), the part being obtained by grinding out brown rice (Koshihikari) successively from the outside using a grinding type rice milling machine (Grain Testing Mill manufactured by SATAKE), and the contents were mixed well.
The mixture was subjected to ultrasonication for 1 minute and 30 seconds. The treated product was left to stand still at 10°C for 12 hours and then centrifuged at 3000 rpm for 10 minutes to perform solid-liquid separation.
An equal amount of ethanol was added to the separated extract supernatant. The contents were mixed well, left to stand still at 10°C for 12 hours and then centrifuged at 3000 rpm for 10 minutes to perform solid-liquid separation.
1 mL of the separated crude extract solution was applied to a column with a diameter of 4 mm and a length of 5 cm (LAS-Serotonin manufactured by J-Oil Mills) packed with a serotonin affinity support (LAS-Serotonin Support manufactured by J-Oil Mills) and fractionated. The conditions of chromatography were as described below. Pure water was run at a flow rate of 0.2 mL/min for 90 minutes (zone 1) to remove unadsorbed components, and subsequently an ammonium acetate solution with a linear gradient from 4 mM to 30 mM was run for 150 minutes (zone 2) to recover the sialic acid-containing compound dissociated from the support. Finally, pure water was run for 90 minutes to regenerate the column (zone 3).
The sialic acid-containing compound was detected by measuring the amount of the compound eluted from the serotonin affinity support in terms of an absorbance at an OD 280 nm. The results are shown in FIG. 8.

Further, the fractionation with the serotonin affinity column was performed twice (twice using 1 mL of the crude extract solution each time). Fractions (zone 2) in which the water-soluble sialic acid-containing compound had been eluted in twice of the fractionation were freeze-dried with a freeze dryer (Freezdryer FD-1 manufactured by EYELA), and the weights of the dried products were measured.

As shown in FIG. 8, it was shown that the recovery of the fractions in which the "water-soluble sialic acid-containing compound" dissociated from the serotonin affinity column had been eluted was attained by running the ammonium acetate solution with a linear gradient from 4 mM to 30 mM through the column (zone 2), after running pure water through the column to remove the component unadsorbed to the serotonin affinity support from the column (zone 1). It was also shown that the sialic acid-containing compound was particularly abundantly eluted in the fractions at around 130 to 160 minutes after the start of elution (start of zone 1) in the zone 2.
In addition, from the fractions (zone 2) obtained by fractionating 2 mL of the crude extract solution (twice by 1 mL) with the serotonin affinity column, 0.5 mg of a dry solid was obtained, and thus, it was shown that 5 mg of the dry solid of the water-soluble sialic acid-containing compound were obtained from 3 g of the flour of the brown rice aleurone layer part as the raw material (20 mL of the crude extract solution were obtained from 3 g of the flour of the brown rice aleurone layer part through the above-mentioned steps).

### Example 2 (Extraction and recovery of sialic acid-containing compounds from rice germ)

15 mL of water were added to 3 g of rice germ (Koshihikari), and the contents were mixed well.
The mixture was subjected to ultrasonication for 1 minute and 30 seconds. The treated product was left to stand still at 10°C for 12 hours and then centrifuged at 3000 rpm for 10 minutes to perform solid-liquid separation.
The separated crude extract solution (0.1 mL) was applied to the column with a diameter of 4 mm and a length of 5 cm (LAS-Serotonin manufactured by J-Oil Mills) packed with the serotonin affinity support (LAS-Serotonin Support manufactured by J-Oil Mills), and fractionated. The conditions of chromatography were as described below. Pure water was run at a flow rate of 0.2 mL/min for 285 minutes to remove the unadsorbed components, and subsequently running of pure water was stopped for 12 hours in order to assure the interaction between the sialic acid-containing compound and the serotonin affinity support. Subsequently, a 30 mM ammonium acetate solution was run for 285 minutes to recover the sialic acid-containing compound dissociated from the support (note that, regeneration of the column was omitted in this example).
The sialic acid-containing compound was detected by measuring the amount of the compound eluted from the serotonin affinity support in terms of an absorbance at OD 280 nm. FIGS. 9 show the results.
Note that, FIG. 9(a) shows a change in absorbance at OD 280 nm at the time of running pure water, and FIG. 9 (b) shows a change in absorbance at OD 280 nm at the time of running the 30 mM ammonium acetate solution.

As shown in FIGS. 9, it was shown that the recovery of the fractions in which the "water-soluble sialic acid-containing compound" dissociated from the serotonin affinity support had been eluted was attained by running pure water through the column (a) to remove the component unadsorbed to the serotonin affinity support, stopping the running of the pure water for 12 hours, and then running the 30 mM ammonium acetate solution through the column (b). It was also shown that the sialic acid-containing compound was particularly abundantly eluted in the fractions at around 30 to 80 minutes after the start of running of the 30 mM ammonium acetate solution through the column.

### Example 3 (Extraction and recovery of sialic acid-containing compounds from polished rice surface layer part)

15 mL of water were added to 3 g of flour derived from a part in the range of 91 to 86% by mass corresponding to a polished rice surface layer part when brown rice was regarded as 100% by mass (polished rice surface layer part having a volume of 5 % by mass), the part being obtained by grinding out brown rice (Koshihikari) successively from the outside using a grinding type rice milling machine (Grain Testing Mill manufactured by SATAKE), and the contents were mixed well.
The mixture was subjected to ultrasonication for 1 minute and 30 seconds. The treated product was left to stand still at 10°C for 12 hours and then centrifuged at 3000 rpm for 10 minutes to perform solid-liquid separation.
2 mL of the separated crude extract solution were applied to a column with a diameter of 26 mm and a length of 20 cm (XK26/20 manufactured by Pharmacia Bioteck) packed with the serotonin affinity support (LAS-Serotonin Support manufactured by J-Oil Mills), in which the support had been packed at a height of 4.6 cm, and was fractionated. The conditions for chromatography were as described below. Pure water was run at a flow rate of 0.2 mL/min for 300 minutes to remove the unadsorbed components (zone 1), and subsequently, the ammonium acetate solution with a linear gradient from 0 M to 1 M was run for 240 minutes to recover the sialic acid-containing compound dissociated from the support (zone 2). Note that, subsequently, the 1M ammonium acetate solution was run for 180 minutes to wash the column (removal of components adsorbed to the column) (zone 3), and finally the linear gradient solution from a 1 M ammonium acetate solution to pure water was run for 60 minutes, and then pure water was run for 180 minutes to regenerate the column (zone 4).
The sialic acid-containing compound was detected by measuring the amount of the compound eluted from the serotonin affinity support in terms of an absorbance at OD 280 nm. FIG. 10 shows the results.
FIG. 10 shows time-dependent changes in absorbance at OD 280 nm of the solutions which run through the serotonin affinity column.

The solutions which had run through the serotonin affinity column by going through the above-mentioned steps were fractionated and recovered as one fraction every 10 minutes to thereby recover 96 fractions in total, which were then freeze-dried.
Representative fractions in the above-mentioned zones 1 to 4 among those fractions were dissolved again in pure water, and the resultant solutions were developed with CH₃Cl/CH₃OH/0.2% CaCl₂=4/4/1 (V/V/V) and separated into individual components on a silica gel plate.
The silica gel plate on which the solutions had been developed was left to stand still under an iodine vapor atmosphere at room temperature for 72 hours to detect all organic compounds. Subsequently, the silica gel plate taken out of the iodine vapor atmosphere was left to stand still for 24 hours to desorb iodine sufficiently, sprayed with resorcinol hydrochloric acid, and heated at 95°C to develop a color, to thereby detect sialic acid-containing compounds. FIGS. 11 show the results.
Note that, in FIGS. 11, FIG. 11(a) shows the all organic compounds detected after the separation of the representative fractions in the zones 1 to 4 by the thin layer chromatography, and FIG. 11(b) shows the sialic acid-containing compounds detected after the separation of the representative fractions in the zones 1 to 4 by the thin layer chromatography.

Further, the fractionation with the serotonin affinity column was performed twice (twice using 2 mL of the crude extract solution each time). Fractions (zone 2) in which the ammonium acetate solution with a linear gradient from 0 M to 1 M had been run in twice of the fractionation were combined and freeze-dried with a freeze-dryer (Freezdryer FD-1 manufactured by EYELA), and the weights of the dried products were measured.

Note that, in FIGS. 11, each lane shows a development on a slica gel plate, wherein lane 1 shows that of the fraction 12 (sample A) eluted at the time between 110 minutes and 120 minutes, lane 2 shows that of the fraction 44 (sample B) eluted at the time between 430 minutes and 440 minutes, lane 3 shows that of the fraction 48 (sample C) eluted at the time between 470 minutes and 480 minutes, lane 4 shows that of the fraction 54 (sample D) eluted at the time between 530 minutes and 540 minutes, lane 5 shows that of the fraction 80 (sample E) eluted at the time between 790 minutes and 800 minutes, and lane 6 shows that of the fraction 89 (sample F) eluted at the time between 880 minutes and 890 minutes, respectively.

As shown in FIG. 10, it was shown that the recovery of the fractions in which the water-soluble sialic acid-containing compound dissociated from the serotonin affinity support had been eluted was attained by running the ammonium acetate solution with a linear gradient from 0 M to 1 M (zone 2: 300 to 540 minutes) through the column, after running pure water through the column to elute the components unadsorbed to the serotonin affinity support from the column (zone 1: 0 to 300 minutes). It was also shown that the water-soluble sialic acid-containing compound dissociated from the serotonin affinity support was eluted in the fractions at around 660 minutes even in the fractions eluted by running the 1 M ammonium acetate solution for washing the column (zone 3: 540 to 720 minutes).
It was also shown that the sialic acid-containing compound was abundantly eluted particularly in the fractions at around 430 minutes (fractions at around 130 minutes after the start of the running of the ammonium acetate solution through the column in zone 2) as a peak in zone 2 and zone 3.
Note that, it is conceivable that the eluted peak of the sialic acid-containing compound was broad in zone 2 and zone 3 probably because the flow rate of the solution was slow relative to the volume of the column used in this example (column having a volume 40 times as large as those in Examples 1 and 2 was used) and thus the eluted peak was diffused.

As shown in FIG. 11B, it was shown that the sialic acid-containing compound was contained in each of the fraction 44 eluted at the time between 430 minutes and 440 minutes (sample B) (lane 2), the fraction 48 eluted at the time between 470 minutes and 480 minutes (sample C) (lane 3), and the fraction 54 eluted at the time between 530 minutes and 540 minutes (sample D) (lane 4).
It was also shown that the sialic acid-containing compound was not contained in the fraction 80 eluted at the time between 790 minutes and 800 minutes (sample E) (lane 5) or the fraction 89 eluted at the time between 880 minutes and 890 minutes (sample F) (lane 6). Those results showed that the sialic acid-containing compound was contained in each of the fractions including the peak in zone 2 in which the ammonium acetate solution was run with a linear gradient from 0 M to 1 M through the column shown in FIG. 10 while the sialic acid-containing compound was not contained in a low peak observed in zone 4 which was a regeneration process of the column.
Note that, as shown by the results of comparison between FIG. 11 (a) and FIG. 11(b), it was shown that the sialic acid-containing compound 'with extremely less contamination with contaminants (content of the contaminants was equal to or less than the detection limit of the all organic compounds detected under the iodine vapor atmosphere)' was recovered from fraction 44 (sample B) eluted at the time between 430 minutes and 440 minutes (lane 2), from fraction 48 (sample C) eluted at the time between 470 minutes and 480 minutes (lane 3), and from fraction 54 (sample D) eluted at the time between 530 minutes and 540 minutes (lane 4).

Those results revealed that the fraction containing the water-soluble sialic acid-containing compound with extremely less contaminants was obtained by extracting from the flour derived from the rice with water as the extraction solvent without using a harmful organic solvent and eluting with ammonium acetate from the serotonin affinity column.

Note that, from the fractions (zone 2) obtained by fractionating 4 mL (twice by 2 mL) of the crude extract solution using the serotonin affinity column, 0.5 to 0.9 mg of a dry solid was obtained, and thus, it was shown that 2.5 to 4.5 mg of a dry solid of the water-soluble sialic acid-containing compound were obtained from 3 g of the flour of the polished rice surface layer part as the raw material (20 mL of the crude extract solution were obtained from 3 g of the flour of the polished rice surface layer part through the above-mentioned steps).

Note that, it was shown that the sialic acid-containing compound was abundantly contained in the fraction 12 (sample A) (lane 1) eluted at the time between 110 minutes and 120 minutes which was the peak in zone 1 in which the components unadsorbed to the serotonin affinity support had been removed from the column by running pure water through the column. This seems to be because the sialic acid-containing compound in an amount which was much more than an adsorbability of the filler of the serotonin affinity column used here for the sialic acid-containing compound was contained in the sample solution applied to the column. Thus, if a larger scale of a column having a sufficient adsorbability for the sialic acid-containing compound, e.g., an industrial column having a volume of about 10 liters or more for the serotonin affinity support is used, the water-soluble sialic acid-containing compound can be separated and recovered more efficiently.

### Test Example 12 (Detection and identification of sialic acid-containing compounds by lectin blot)

15 mL of ethylene glycol were added to 3 g of rice germ (Koshihikari), and the contents were mixed well. The mixture was subjected to ultrasonication using an ultrasonic apparatus (B-42J Ultrasonic Cleaner manufactured by BRANSON) for 1 minute and 30 seconds. The treated product was left to stand still at 10°C for 12 hours and then centrifuged at 3000 rpm for 10 minutes to perform solid-liquid separation (sample 12). The "centrifuged supernatant of the extract solution from the rice germ with ethylene glycol" (sample 12) obtained by the above-mentioned steps was developed with CH₃Cl/CH₃OH/0.2% CaCl₂=4/4/1 (V/V/V) or CH₃Cl/CH₃OH/water=4/4/1 (V/V/V) and separated into individual components on a silica gel plate. The silica gel plate was then dried with cold air.
The silica gel plate was immersed in a solution of poly(isobutyl methacrylate) in n-hexane/CH₃Cl=9/1 (V/V)
(concentration: 2 g/500 mL) for 30 seconds, and dried with cold air.

Then, the silica gel plate treated with poly(isobutyl methacrylate) was immersed in a solution of TRITIC fluorescence-labeled MAA lectin (manufactured by E. Y. Labolatory) diluted to 100 times with a 0.01 M phosphate buffer (pH 7.2 to 7.4) containing 0.15 M sodium chloride and 1% BSA or a solution of TRITIC fluorescence-labeled SNA-I lectin (manufactured by E. Y. Labolatory) diluted to 100 times with a 0.01 M phosphate buffer (pH 7.2 to 7.4) containing 0.15 M sodium chloride and 1% BSA in a plastic container, and shaken at room temperature for 48 hours to perform a lectin binding reaction.
Note that the 'MAA lectin' is a lectin which is specifically bound to an "N-acetylneuraminic acid α (2→3) Gal" structure, and the 'SNA-I lectin' is a lectin which is specifically bound to an "N-acetylneuraminic acid α (2→6) Gal" or "N-acetylneuraminic acid α (2→6) GalNAc" structure.
After the completion of the lectin binding reaction, the silica gel plate treated with poly(isobutyl methacrylate) was washed to remove the fluorescent lectin which was unbound to the objective compound. Subsequently, the sialic acid-containing compound was detected by observing a portion where fluorescence was observed, i.e., a site where the lectin specifically bound to the sialic acid was present. The results are shown by each combination of detection conditions in FIGS. 12A to 12D.

Note that FIG. 12A shows the results of a reaction of the TRITIC fluorescence-labeled MAA lectin with the silica gel plate developed with CH₃Cl/CH₃OH/0.2% CaCl₂=4/4/1 (V/V/V).
FIG. 12B shows the results of a reaction of the TRITIC fluorescence-labeled MAA lectin with the silica gel plate developed with CH₃Cl/CH₃OH/water=4/4/1 (V/V/V).
FIG. 12C shows the results of a reaction of the TRITIC fluorescence-labeled SNA-I lectin with the silica gel plate developed with CH₃Cl/CH₃OH/0.2% CaCl₂=4/4/1 (V/V/V).
FIG. 12D shows the results of a reaction of the TRITIC fluorescence-labeled SNA-I lectin with the silica gel plate developed with CH₃Cl/CH₃OH/water=4/4/1 (V/V/V).
In each of FIGS. 12A to 12D, the left-hand photograph is an image of the silica gel plates captured before the lectin binding reaction, and the right-hand photograph is an image of the silica gel plates captured after the lectin binding reaction.

As shown in FIGS. 12A to 12D, it was observed that portions which did not emit light before the fluorescent lectin binding reaction (left-hand photograph in each figure) emitted light after the fluorescent lectin binding reaction (right-hand photograph in each figure) (portions indicated by arrows). The light-emitting portion on the right-hand photograph in each figure indicates that the fluorescence-labeled MAA lectin or SNA-I lectin was specifically bound to the compound having the structure specific for N-acetylneuraminic acid as described above.
Specifically, FIGS. 12A and 12B (detection results by MAA lectin) showed that the compound having the "N-acetylneuraminic acid α (2→3) Gal" structure was present in each of the compounds developed on the silica gel plate and shown by the arrows.
In addition, FIGS. 12C and 12D (detection results by SNA-I lectin) showed that the compound having the "N-acetylneuraminic acid α (2→6) Gal" structure or "N-acetylneuraminic acid α (2→6) GalNAc" structure was present in each of the compounds developed on the silica gel plate and shown by the arrows.
Therefore, the results of this test example showed that the 'compound containing sialic acid (N-acetylneuraminic acid)' was present in the solution extracted from the rice germ with ethylene glycol.

### Test Example 13 (Detection of sialic acid-containing compounds from crude extract solution of cooked rice)

"Rice excluding a part corresponding to the range of 100 to 96% by mass (brown rice outermost layer part) when brown rice was regarded as 100% by mass and including the remaining germ and aleurone layer" (in other words, rice having a volume of 96 % by mass and including the remaining germ and aleurone layer, obtained by grinding out the brown rice outermost layer part), obtained by grinding out brown rice (Koshihikari) from the outside using a brush type rice milling machine (HRG-122 manufactured by Minoru Industry Co., Ltd.) was immersed in water at 20°C (samples 13-1 and 13-2) or 25°C (samples 13-3, 13-4) and left to stand still for 6 hours. The rice was lightly washed three times, 2 parts by mass of water were added to 2 parts by mass of the rice, and the mixture was cooked with a home rice cooker. 15 mL of water were added to 3 g of the cooked rice, and the contents were mixed well.
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.
The separated supernatants were developed with CH₃Cl/CH₃OH/water=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 13 shows the results.

Note that, in FIG. 13, 2 µL of a crude extract solution (sample 13-1) extracted in the above-mentioned steps using water as the extraction solvent from the cooked rice obtained by immersing the 'rice excluding the brown rice outermost layer and including the germ and the aleurone layer' in water at 20°C, leaving the rice to stand still for 6 hours, and then cooking the rice were developed in lane 1 on the silica gel plate. In lane 2, 4 µL of the same sample as that in lane 1 (sample 13-2) was developed on the silica gel plate.
In lane 3, 2 µL of a crude extract solution (sample 13-3) extracted in the above-mentioned steps using water as the extraction solvent from the cooked rice obtained by immersing the 'rice excluding the brown rice outermost layer and including the germ and the aleurone layer' in water at 25°C, leaving the rice to stand still, and then cooking the rice were developed on the silica gel plate. In lane 4, 4 µL of the same sample as that in lane 3 (sample 13-4) were developed on the silica gel plate.

As shown by the results in FIG. 13, it was shown that even if the 'rice excluding the brown rice outermost layer and including the germ and the aleurone layer' was cooked, the crude extract solution containing the water-soluble sialic acid-containing compound was obtained by extracting from the cooked rice with water.

### Test Example 14 (Detection of sialic acid-containing compounds from crude extract solution using glycerol)

15 mL of 100% (v/v) glycerol were added to 3 g of flour obtained by milling wheat (Norin 61) whole grains or barley (Sachiho Golden, Betzes, Daishimochi, Ichibanboshi, Karasugane No. 1) whole grains with a mill (Ultracentrifugal Mill manufactured by MRK-RETSCH), and the contents were mixed well (samples 14-1 to 14-6).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
The separated crude extract solutions were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 14 shows the results.

Note that, in FIG. 14, each lane shows a development on a slica gel plate, wherein lane 1 shows that of the crude extract solution obtained from the wheat Norin 61 whole grain flour using 100% (v/v) glycerol as the extraction solvent in the above-mentioned steps (sample 14-1), lane 2 shows that of the crude extract solution obtained from the barley Sachiho Golden whole grain flour using 100% (v/v) glycerol as the extraction solvent in the above-mentioned steps (sample 14-2), lane 3 shows that of the crude extract solution obtained from the barley Betzes whole grain flour using 100% (v/v) glycerol as the extraction solvent in the above-mentioned steps (sample 14-3), lane 4 shows that of the crude extract solution obtained from the barley Daishimochi whole grain flour using 100% (v/v) glycerol as the extraction solvent in the above-mentioned steps (sample 14-4), lane 5 shows that of the crude extract solution obtained from the barley Ichibanboshi whole grain flour using 100% (v/v) glycerol as the extraction solvent in the above-mentioned steps (sample 14-5), and lane 6 shows that of the crude extract solution obtained from the barley Karasugane No. 1 whole grain flour using 100% (v/v) glycerol as the extraction solvent in the above-mentioned steps (sample 14-6), respectively.

As shown by the results in FIG. 14, it was shown that the crude extract solution containing the water-soluble sialic acid-containing compound was obtained by extracting from the wheat whole grain flour or the barley whole grain flour using 100% (v/v) glycerol as the extraction solvent.

### Test Example 15 (Detection of sialic acid-containing compounds from each of crude extract solutions of flours and processed products derived from various cereals)

15 mL of water were added to 3 g of flour obtained by milling wheat (Norin 61) whole grains or the barley (Sachiho Golden, Betzes, Daishimochi, Ichibanboshi, Karasugane No. 1) whole grains with a mill (Ultracentrifugal Mill manufactured by MRK-RETSCH), and the contents were mixed well (samples 15-1 to 15-6). In addition, 15 mL of water were added to 3 g of the wheat (Norin 61) short or barley (Sachiho Golden) short, and the contents were mixed well (samples 15-7 and 15-8). Also, 15 mL of water were added to 3 g of flour obtained by milling rice (Hatsushimo) germ with the mill (Ultracentrifugal Mill manufactured by MRK-RETSCH), and the contents were mixed well (sample 15-9).
Those mixtures were subjected to ultrasonication using an ultrasonic apparatus (B-42J Ultrasonic Cleaner manufactured by BRANSON) for 1 minute and 30 seconds. Those treated products were immediately centrifuged at 1000 rpm for 10 minutes to perform solid-liquid separation.
The separated crude extract solutions were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 15 shows the results.

Note that, in FIG. 15, each lane shows a development on a slica gel plate, wherein lane 1 shows that of the crude extract solution obtained from the wheat Norin 61 whole grain flour using water as the extraction solvent in the above-mentioned steps (sample 15-1), lane 2 shows that of the crude extract solution obtained from the barley Sachiho Golden whole grain flour using water as the extraction solvent in the above-mentioned steps (sample 15-2), lane 3 shows that of the crude extract solution obtained from the barley Betzes whole grain flour using water as the extraction solvent in the above-mentioned steps (sample 15-3), lane 4 shows that of the crude extract solution obtained from the barley Daishimochi whole grain flour using water as the extraction solvent in the above-mentioned steps (sample 15-4), lane 5 shows that of the crude extract solution obtained from the barley Ichibanboshi whole grain flour using water as the extraction solvent in the above-mentioned steps (sample 15-5), lane 6 shows that of the crude extract solution obtained from the barley Karasugane No. 1 whole grain flour using water as the extraction solvent in the above-mentioned steps (sample 15-6), lane 7 shows that of the crude extract solution obtained from the wheat Norin 61 short using water as the extraction solvent in the above-mentioned steps (sample 15-7), lane 8 shows that of the crude extract solution obtained from the barley Sachiho Golden short using water as the extraction solvent in the above-mentioned steps (sample 15-8), and lane 9 shows that of the crude extract solution obtained from the milled rice (Hatsushimo) germ flour using water as the extraction solvent in the above-mentioned steps (sample 15-9), respectively.

As shown by the results in FIG. 15, it was shown that the crude extract solution containing the water-soluble sialic acid-containing compound was obtained by extracting using water as the extraction solvent from the wheat or barley whole grain flour or short, or the rice germ.
It was shown that one specific type of the sialic acid-containing compound was extracted from the rice germ at higher concentration compared with the wheat and barley whole grain flours and short. It was also shown that multiple types of the sialic acid-containing compounds which were not contained in the rice germ could be extracted from the wheat and the barley. Concerning the wheat and the barley, it was shown that a higher concentration of the sialic acid-containing compound was extracted using the short as the extraction raw material rather than using the whole grain flours.

### Test Example 16 (Influence of acid type on extraction)

15 mL of water, 11.83% (2 N) acetic acid, or 7.06% (2 N) hydrochloric acid were added to 3 g of wheat Norin 61 short, and the contents were mixed well. Those mixtures were subjected to ultrasonication using an ultrasonic apparatus (B-42J Ultrasonic Cleaner manufactured by BRANSON) for 1 minute and 30 seconds. Those treated products were shaken at 80°C for 3 hours (sample 16-1 to 16-3) and then centrifuged at 1000 rpm for 10 minutes to perform solid-liquid separation.
Also, 15 mL of water were added to 3 g of the wheat Norin 61 short, and the contents were mixed well. The mixture was subjected to ultrasonication using an ultrasonic apparatus (B-42J Ultrasonic Cleaner manufactured by BRANSON) for 1 minute and 30 seconds. The treated product was centrifuged at 1000 rpm for 10 minutes immediately (Sample 16-4) or after being left to stand still at 10 °C for 2.85 h (about 3 hours) (Sample 16-8), to perform solid-liquid separation.
The separated crude extract solutions were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby sialic acid-containing compounds. FIG. 16 shows the results.

Note that, in FIG. 16, each lane shows a development on a slica gel plate, wherein lane 1 shows that of the crude extract solution obtained by extracting from the wheat Norin 61 short using water as the extraction solvent, then ultrasonication followed by shaking at 80°C for 3 hours and subsequent centrifugation thereof (sample 16-1), lane 2 shows that of the crude extract solution obtained by extracting from the wheat Norin 61 short using 2 N acetic acid as the extraction solvent, then ultrasonication followed by shaking at 80°C for 3 hours and subsequent centrifugation thereof (sample 16-2), and lane 3 shows that of the crude extract solution obtained by extracting from the wheat Norin 61 short using 2N hydrochloric acid as the extraction solvent, then ultrasonication followed by shaking at 80°C for 3 hours and subsequent centrifugation thereof (sample 16-3), respectively.
Also, the crude extract solution obtained by extracting from the wheat Norin 61 short using water as the extraction solvent and then ultrasonication immediately followed by centrifugation thereof (sample 16-4), and the crude extract solution obtained by extracting from the wheat Norin 61 short using water as the extraction solvent and then ultrasonication followed by being left to stand still at 10°C for about 3 hours and subsequent centrifugation thereof (sample 16-8) were developed in lanes 4 and 8, respectively on the silica gel plate.
In addition, a sialic acid-containing compound authentic GD1a (manufactured by SIGMA), a sialic acid-containing compound authentic GT1b (manufactured by SIGMA), and a sialic acid-containing compound authentic N-acetylneuraminic acid (manufactured by SIGMA) were developed in lanes 5, 6, and 7, respectively on the silica gel plate.

As shown by the results in FIG. 16, it was shown that the sialic acid-containing compound which was different from those extracted using water and migrated to higher positions on the silica gel plate was obtained by treatment with acetic acid or hydrochloric acid at 80°C for about 3 hours. Those results are thought to be because 'the molecular weight of the sialic acid-containing compound was reduced or the sialic acid-containing compound was fragmented' by the treatment with the acid plus heat to thereby reduce its polarity.
That is, it was suggested that the treatment with the acid plus heat was able to reduce the molecular weight or cause the fragmentation to enhance easily-soluble property (enhance the elution efficiency).

### Test Example 17 (Influence of extraction time on extraction at room temperature)

10 mL of water were added to 2 g of wheat (Shirasagikomugi) short, and the contents were mixed well. The mixture was subjected to ultrasonication using an ultrasonic apparatus (B-42J Ultrasonic Cleaner manufactured by BRANSON) for 1 minute and 30 seconds. The treated product was centrifuged at 1000 rpm for 10 minutes immediately (sample 17-1) or after shaking at 25°C for 1 hour (sample 17-2), 4 hours (sample 17-3), 12 hours (sample 17-4), or 24 hours (sample 17-5) to perform solid-liquid separation.
The separated crude extract solutions were developed with CH₃Cl/acetone/CH₃OH/acetic acid/water/pyridine=75/45/30/25/5/20 (V/V/V/V/V/V) and separated into individual components on a silica gel plate. The silica gel plate was sprayed with resorcinol hydrochloric acid and heated at 95°C to develop a color, to thereby detect sialic acid-containing compounds. FIG. 17 shows the results.

Note that, in FIG. 17, each lane shows a development on a slica gel plate, wherein lane 1 shows that of the crude extract solution obtained immediately after the ultrasonication after extracting from the wheat (Shirasagikomugi) short with water as the extraction solvent in the above-mentioned steps (sample 17-1), lane 2 shows that of the crude extract solution obtained after the ultrasonication followed by shaking at 25°C for 1 hour after extracting from the wheat (Shirasagikomugi) short with water as the extraction solvent in the above-mentioned steps (sample 17-2), lane 3 shows that of the crude extract solution obtained after the ultrasonication followed by shaking at 25°C for 4 hours after extracting from the wheat (Shirasagikomugi) short with water as the extraction solvent in the above-mentioned steps (sample 17-3), lane 4 shows that of the crude extract solution obtained after the ultrasonication followed by shaking at 25°C for 12 hours after extracting from the wheat (Shirasagikomugi) short with water as the extraction solvent in the above-mentioned steps (sample 17-4), and lane 5 shows that of the crude extract solution obtained after the ultrasonication followed by shaking at 25°C for 24 hours after extracting from the wheat (Shirasagikomugi) short with water as the extraction solvent in the above-mentioned steps (sample 17-5), respectively.

As shown by the results in FIG. 17, it was shown that the color-developed bands close to an origin were shifted to bands developed on upper positions by immersing the wheat short in water at 25°C (for 4 hours or more, particularly 12 hours or more). The result was obtained probably because 'the molecular weight of the sialic acid-containing compound was reduced or the sialic acid-containing compound was fragmented' to thereby reduce its polarity by an action of the endogenous enzyme.
That is, it was suggested that a reduction in molecular weight of the sialic acid-containing compound in the wheat short or the fragmentation of the sialic acid-containing compound (a change in composition of the sialic acid-containing compound) to enhance the easily-soluble property (enhance the elution efficiency) was attained without the addition of a specific enzyme.

### Test Example 18 (Detection and identification of sialic acid-containing compounds by lectin blot from crude extract solution of each cereal bran)

15 mL of water were added to 3 g of rice (Hitomebore) bran, wheat (Nishinochikara) bran, or barley (Mannenboshi) bran, and the contents were mixed well.
Those mixtures were subjected to ultrasonication using an ultrasonic apparatus (B-42J Ultrasonic Cleaner manufactured by BRANSON) for 1 minute and 30 seconds. Those treated products were immediately centrifuged at 1000 rpm for 10 minutes to perform solid-liquid separation (samples 18-1 to 18-3).
The crude extract solution obtained by the above-mentioned steps was developed with CH₃Cl/CH₃OH/0.2% CaCl₂=4/4/1 (V/V/V) and separated into individual components on a silica gel plate. The silica gel plate was then dried with cold air.
The silica gel plate was immersed in a solution of poly(isobutyl methacrylate) in n-hexane/CH₃Cl=9/1 (v/v) (concentration: 2 g/500 mL) for 30 seconds, and dried with cold air.

Then, the silica gel plate treated with poly(isobutyl methacrylate) was immersed in a solution of TRITIC fluorescence-labeled MAA lectin (manufactured by E. Y. Labolatory) diluted to 100 times with a 0.01 M phosphate buffer (pH 7.2 to 7.4) containing 0.15 M sodium chloride and 1% BSA, and shaken at room temperature for 24 hours to perform the lectin binding reaction.
Note that the 'MAA lectin' is a lectin which is specifically bound to an "N-acetylneuraminic acid α (2→3) Gal" structure.
After the completion of the lectin binding reaction, the silica gel plate treated with poly(isobutyl methacrylate) was washed to remove the fluorescent lectin which was unbound to the objective compound. Subsequently, the sialic acid-containing compound was detected by observing a portion where fluorescence was observed, i.e., a site where the lectin specifically bound to the sialic acid was present. FIG. 18 shows the results.

Note that FIG. 18 shows the results of a reaction of the TRITIC fluorescence-labeled MAA lectin with the silica gel plate developed with CH₃Cl/CH₃OH/0.2% CaCl₂=4/4/1 (V/V/V). That is, the left-hand photograph is an image of the silica gel plate captured before the lectin binding reaction, and the right-hand photograph is an image of the silica gel plate captured after the lectin binding reaction.
In FIG. 18, each lane shows a development on a slica gel plate, wherein lane 1 shows that of the crude extract solution obtained by extracting from the rice (Hitomebore) bran with water as the extraction solvent in the above-mentioned steps (sample 18-1), lane 2 shows that of the crude extract solution obtained by extracting from the wheat (Nishinochikara) bran with water as the extraction solvent in the above-mentioned steps (sample 18-2), and lane 3 shows that of the crude extract solution obtained by extracting from the barley (Mannenboshi) bran with water as the extraction solvent in the above-mentioned steps (sample 18-3), respectively.

As shown in FIG. 18, it was observed that in the portion in which light was not emitted before the fluorescent lectin binding reaction (left-hand photograph in the figure), light was emitted (portion indicated by arrows) after the fluorescent lectin binding operation (right-hand photograph in the figure). The light-emitting portion in the right-hand photograph in the figure indicates that the fluorescence-labeled MAA lectin was specifically bound to the compound having the structure specific for N-acetylneuraminic acid described above.
To be specific, the photograph indicates that the compound having the "N-acetylneuraminic acid α (2→3) Gal" structure is present.
Therefore, the results in this test example showed that the 'compound containing sialic acid (N-acetylneuraminic acid)' was present in the extract solution of the rice bran, the wheat bran, or the barley bran extracted with water.

### Industrial Applicability

According to the present invention, there can be provided the water-soluble sialic acid-containing compound from raw materials derived from plants, which have no risk of contamination with pathogens affecting animals and are safe upon ingestion, particularly from seeds of cereal or seeds of bean or processed products thereof.
Also according to the present invention, there can be provided the water-soluble sialic acid-containing compound from rice bran (red bran or white bran), wheat bran, barley bran, soybean waste, irregular adzuki beans, and residue after extracting starch from corn, which are wastes derived from the plants.
Further, the sialic acid-containing compound derived from the plant, which is the article of the present invention, can be utilized for functional foods, high value-added cosmetics, and pharmaceutical raw materials each containing sialic acid.

## Claims

1. An extraction method for a water-soluble sialic acid-containing compound, the method comprising:
crudely extracting a water-soluble component with water, polyol, or water containing either an acid or an alkali or polyol from a plant body or a processed product of the plant body; and
separating and recovering the water-soluble sialic acid-containing compound from the resultant crude extract solution with a column packed with a serotonin affinity support.

2. The extraction method for a water-soluble sialic acid-containing compound according to claim 1, wherein ultrasonication is performed in the step of crudely extracting the water-soluble component with water, the polyol, or water containing either the acid or the alkali or the polyol.

3. The extraction method for a water-soluble sialic acid-containing compound according to claim 1 or 2, wherein ethanol is added to precipitate and remove contaminants after the step of crudely extracting the water-soluble component with water, the polyol, or water containing either the acid or the alkali or the polyol.

4. The extraction method for a water-soluble sialic acid-containing compound according to any one of claims 1 to 3, wherein the plant body includes seeds of cereal or seeds of bean.

5. A water-soluble sialic acid-containing compound, which is obtained by the extraction method according to any one of claims 1 to 4.
